# EUROPEAN PATENT SPECIFICATION

(11) **EP 4 655 601 B1**
(45) Date of publication and mention of the grant of the patent: **12.08.2026**
(21) Application number: 23718242.3
(22) Date of filing: 06.04.2023
(51) Int. Cl.: G01R 31/62

(54) **THERMOCHEMICAL DIGITAL TWIN OF ELECTRICAL TRANSFORMER**
THERMOCHEMISCHER DIGITALER ZWILLING EINES ELEKTRISCHEN TRANSFORMATORS
JUMEAU NUMÉRIQUE THERMOCHIMIQUE DE TRANSFORMATEUR ÉLECTRIQUE

(30) Priority: 24.01.2023 US 202363440843 P
(43) Date of publication of application: 03.12.2025
(73) Proprietor: Hitachi Energy Ltd, 8050 Zürich (CH)
(72) Inventor: SBRAVATI, Alan, Raleigh, North Carolina 27610 (US); CHEIM, Luiz V., Raleigh, North Carolina 27603 (US); FRIMPONG, George K., Raleigh, North Carolina 27610 (US)
(74) Representative: Vossius & Partner Patentanwälte Rechtsanwälte mbB
(86) International application number: PCT/EP2023/059172
(87) International publication number: WO 2024/156373

(56) References cited:
- CN-A- 115 563 897
- US-A1- 2022 137 612
- WANG LIANG ET AL: "Research on digital twin modeling method of transformer temperature field based on POD", ENERGY REPORTS, vol. 9, 24 June 2022 (2022-06-24), pages 299 - 307, XP093090291, ISSN: 2352-4847, Retrieved from the Internet <URL:https://pdf.sciencedirectassets.com/311225/1-s2.0-S2352484722X00188/1-s2.0-S2352484723002470/main.pdf?X-Amz-Security-Token=IQoJb3JpZ2luX2VjEMz//////////wEaCXVzLWVhc3QtMSJIMEYCIQDt/mUsYLrdNevQE3csp/o18OCIbOVmCcZt08FQmFQ2PQIhALUaiqHvwARCblu3gYvXjL3jPUfOXMouPbXXxuR6audqKrwFCNX//////////wEQBRoMMDU5MDAzN> [retrieved on 20231011], DOI: 10.1016/j.egyr.2023.03.010

## Description

### CROSS-REFERENCE TO RELATED APPLICATIONS

This application claims priority to U.S. Provisional Patent App. No. 63/440,843, filed on January 24, 2023.

### BACKGROUND

### Field of the Invention

The embodiments described herein are generally directed to a digital twin of an electrical transformer, and, more particularly, to modeling thermochemical degradation in a digital twin of an electrical transformer.

### Description of the Related Art

The residual life of an electrical transformer is commonly associated with the life of the solid insulation within the electrical transformer. While the temperature is the most relevant parameter in calculating the life consumption of the electrical transformer, the influence of water and oxygen content cannot be denied, especially at partial loading conditions. For example, water negatively affects dielectric capacity and degrades the insulation. However, water is a natural byproduct of aging of the solid insulation (e.g., paper or other cellulose-based material) and the insulating liquid (e.g., mineral oil, liquid ester, etc.). In fact, water removal is one of the most common reasons for maintenance interventions with electrical transformers.

One challenge in assessing electrical transformers within power networks is that it is impossible to measure many of the parameters that are relevant to the electrical transformer's performance and health. A sensor will interfere with field distribution, and output biased measurements. Consequently, a sensor cannot be used to measure the water content in the solid insulation inside an electrical transformer. Furthermore, even if a sample is taken from a critical location of the insulation, that sample will no longer represent the same condition after it has cooled down to ambient temperature and/or been immersed in any liquid that is not the insulating liquid inside the electrical transformer. The document "Research on digital twin modeling method of transformer temperature field based on POD" by WANG LIANG ET AL in ENERGY REPORTS, vol. 9, 24 June 2022 (2022-06-24), pages 299-307, XP093090291,ISSN: 2352-4847, teaches to use a digital twin to model the temperature field of a transformer using proper orthogonal decomposition for calculating the temperature rise in a transformer.

Thus, it is infeasible to directly measure the water content in the solid insulation of an electrical transformer. Accordingly, the present disclosure is directed toward modeling thermal degradation in a digital twin of an electric transformer, such that the water content in the solid insulation can be estimated. The present disclosure is also directed to one or more other problems discovered by the inventors.

### SUMMARY

Systems, methods, and non-transitory computer-readable media are disclosed for modeling thermochemical degradation in a digital twin of an electric transformer. One aspect of one or more disclosed embodiments is the estimation of the total water content in insulation of the electrical transformer based on both the diffusion, between the insulating liquid and the insulation, and the degradation of the insulation. A further aspect is the logical division of the electrical transformer into a plurality of sections, such that the total water content in the insulation of each section can be estimated. An even further aspect is the utilization of artificial intelligence to adjust the total water content in the electrical transformer, in order to account for leakage, hydrolysis, and/or other chemical processes.

In an embodiment, a method, for modeling thermochemical degradation in a digital twin of an electrical transformer, comprises using at least one hardware processor to, for each of one or more time intervals: determine a total water content in insulating liquid of the electrical transformer; for each of a plurality of sections of at least one winding in the electrical transformer, logically divided along at least one axis of the at least one winding, estimate a temperature of the section based on a loading of the electrical transformer and a temperature gradient along the at least one axis, estimate a diffusion-related water content in insulation of the at least one winding in the section, resulting from diffusion, based on the estimated temperature and the total water content in the insulating liquid, estimate a degradation-related water content, based on degradation of a material of the insulation, and estimate a total water content within the insulation of the at least one winding in the section based on the diffusion-related water content and the degradation-related water content; and estimate a total water content in the electrical transformer based on the total water content in the insulating liquid and the total water content within the insulation of the at least one winding in all of the plurality of sections.

The method may further comprise using the at least one hardware processor to, for each of the one or more time intervals, determine an average temperature of the insulating liquid for each of a plurality of time instants spanning the time interval. The plurality of sections may comprise: at least one lower stack that represents a first assembling element at a first end of the at least one winding along the longitudinal axis; at least one upper stack that represents a second assembling element at a second end of the at least one winding that is opposite the first end of the at least one winding along the longitudinal axis; and one or more coil sections that each represents at least a portion of at least one coil between the first end and the second end of the at least one winding. The temperature gradient may comprise an axial temperature gradient along an axial axis and a radial temperature gradient along a radial axis, and estimating the temperature of each of the plurality of sections may comprise, for each of the plurality of time instants: calculating a localized temperature of the at least one lower stack, based on the average temperature of the insulating liquid and the axial temperature gradient calculated for the time instant; calculating a localized temperature of the at least one upper stack based on the average temperature of the insulating liquid and the axial temperature gradient calculated for the time instant; and calculating a localized temperature for each of the one or more coil sections, based on the temperature of the at least one lower stack or at least one upper stack, the loading, and the radial and axial temperature gradients. The one or more coils sections may be a plurality of coil sections, and the temperature for one of the plurality of coil sections that is closest to the second end may be calculated further based on a hot-spot factor or heat-dissipation factor associated with the electrical transformer.

Estimating the diffusion-related water content may comprise, for each of a plurality of time instants spanning the time interval, calculating a water content diffused into the insulation based on a time constant for diffusion of water into the material of the insulation. For each of the plurality of time instants, the water content diffused into the insulation may be calculated as: ${W}_{diffusion} = \left({W}_{equilibrium}-{W}_{initial}\right) ⋅ \left(1-{e}^{- \frac{t}{{τ}_{p}}}\right) + {W}_{initial}$ wherein *W_{diffusion}* is the water content diffused into the insulation at time instant *t, W_{equilibrium}* is a water content in the insulation at an equilibrium condition, *Wᵢₙᵢₜᵢₐₗ* is a water content in the insulation at a time instant immediately preceding time instant *t, e* is Euler's number, and *τₚ* is the time constant.

The material of the insulation may be cellulose-based. The degradation-related water content in the insulation may be estimated based on a water generation rate from scissions of cellulose chains in the cellulose-based material. The water generation rate may be calculated as: ${W}_{rate} = {\int }_{0}^{k} {2}^{x + 1} dx ⋅ \frac{{Mol}_{water}}{DP ⋅ {Mol}_{glucose}} ⋅ \frac{1}{180 , 000}$ wherein *Wᵣₐₜₑ* is an amount of water generated per hour, *k* is a number of scissions, *Mol_{water}* is a molar mass of water, *DP* is a degree of polymerization of the material of the insulation, and *Mol_{glucose}* is a molar mass of a monomer of glucose.

The method may further comprise using the at least one hardware processor to, for each of the one or more time intervals, for each of the plurality of sections of the at least one winding in the electrical transformer, calculate a measure of aging for the section based on the total water content in the insulation; and determine an overall measure of aging of the electrical transformer based on the measures of aging for the plurality of sections. The measure of aging for each of the plurality of sections may be further based on one or both of an oxygen content in the insulation or an acidity of the insulating liquid. Calculating the measure of aging for each of the plurality of sections may comprise: determining a rated aging parameter associated with the material of the insulation; interpolating an aging parameter of the section based on the total water content in the insulation of the at least one winding in the section; and calculating an aging ratio between the rated aging parameter and the interpolated aging parameter. Calculating the measure of aging for each of the plurality of sections may further comprise: determining an activation energy at rated conditions associated with the material of the insulation; interpolating an activation energy of the material of the insulation; calculating an exponentiation factor based on the activation energy at rated conditions and the interpolated activation energy; and determining the measure of aging for the section based on the aging ratio and the exponentiation factor. The measure of aging for each of the plurality of sections may be calculated as: $V = \frac{A}{{A}_{r}} ⋅ {e}^{\frac{1}{R} \times \left(\frac{{E}_{r}}{{θ}_{h , r} + 273}-\frac{E}{{θ}_{h} + 273}\right)}$ wherein *V* is the measure of aging, *A* is the interpolated aging parameter, *Aᵣ* is the rated aging parameter, *e* is Euler's number, *R* is a molar gas constant, *Eᵣ* is the activation energy at rated conditions, *E* is the interpolated activation energy, *θ_{h,r}* is a hot-spot-to-top-oil temperature at a rated load, and *θₕ* is a hot-spot temperature.

Determining the total water content in the insulating liquid may comprise deriving the total water content in the insulating liquid from an output of at least one sensor in the electrical transformer, and the method may further comprise using the at least one hardware processor to, at least once for each of the one or more time intervals: calculate a deviation between an actual value of the total water content in the insulating liquid, derived from the output of the at least one sensor, and one or more estimated values of the total water content in the insulating liquid output by an artificial intelligence (AI) model of the digital twin; and determine an adjustment parameter based on the deviation, wherein the estimate of the total water content in the transformer is further based on the adjustment parameter.

The method may further comprise using the at least one hardware processor to estimate a risk of dielectric failure of the insulation of the at least one winding based on the estimated total water content within the insulation.

The method may further comprise using the at least one hardware processor to estimate a likelihood of microbubbles on a surface of the insulation of the at least one winding based on the estimated total water content within the insulation.

The method may further comprise using the at least one hardware processor to: estimate a breakdown voltage of the insulating liquid based on the total water content in the insulating liquid; and when the estimated breakdown voltage satisfies a threshold, generate an alert.

It should be understood that any of the features in the methods above may be implemented individually or with any subset of the other features in any combination. Thus, to the extent that the appended claims would suggest particular dependencies between features, disclosed embodiments are not limited to these particular dependencies. Rather, any of the features described herein may be combined with any other feature described herein, or implemented without any one or more other features described herein, in any combination of features whatsoever.

In addition, any of the methods, described above and elsewhere herein, may be embodied, individually or in any combination, in executable software modules of a processor-based system, such as a server, and/or in executable instructions stored in a non-transitory computer-readable medium.

### BRIEF DESCRIPTION OF THE DRAWINGS

The details of the present invention, both as to its structure and operation, may be gleaned in part by study of the accompanying drawings, in which like reference numerals refer to like parts, and in which:
FIG. 1 illustrates an example infrastructure, in which one or more of the processes described herein, may be implemented, according to an embodiment;
FIG. 2 illustrates an example processing system, by which one or more of the processes described herein, may be executed, according to an embodiment;
FIG. 3 illustrates a schematic representation of a discretized electrical transformer, according to an embodiment;
FIG. 4 illustrates a process for modeling thermochemical degradation in a digital twin of an electrical transformer, according to an embodiment;
FIG. 5 illustrates a chemical representation of two monomers of glucose, according to an example;
FIG. 6 illustrates the water content in insulating liquid and insulation in a simulated electrical transformer, according to a tested implementation of an embodiment;
FIG. 7 illustrates water generation, from cellulose degradation, in a simulated electrical transformer, according to a tested implementation of an embodiment; and
FIG. 8 illustrates a measure of aging for a simulated electrical transformer, according to a tested implementation of an embodiment.

### DETAILED DESCRIPTION

In an embodiment, systems, methods, and non-transitory computer-readable media are disclosed for modeling thermochemical degradation in a digital twin of an electric transformer. After reading this description, it will become apparent to one skilled in the art how to implement the invention in various alternative embodiments and alternative applications. However, although various embodiments of the present invention will be described herein, it is understood that these embodiments are presented by way of example and illustration only, and not limitation. As such, this detailed description of various embodiments should not be construed to limit the scope or breadth of the present invention as set forth in the appended claims.

Traditional methods of estimating thermochemical degradation of an electric transformer consist of measuring the water content in the insulating liquid (e.g., by sensor or manual sampling), testing the electrical transformer during scheduled outages (e.g., to measure power factor, insulation resistance, dielectric frequency response, etc.), or draining the insulating liquid and measuring the dew point of the insulation after twenty-four hours in contact with dry air.

The least invasive of these is monitoring the water content in the insulating liquid via a sensor. However, such monitoring is not trivial. Neither the loading nor the ambient temperatures remain constant long enough for the system to reach an equilibrium condition. Consequently, any measurement is only a snapshot of a transient condition. The traditional procedure "corrects" the water content to a condition at a reference temperature of 20° Celsius (C). The water saturation in mineral oil approximately doubles for every 40°C of temperature increase, over the temperature reference of 20°C. Thus, a reading in parts per million (ppm) has a completely different meaning when the temperature of the mineral oil is at 20°C than when the temperature of the mineral oil is at 100°C. The typical solution to this is to check the water content, relative to saturation, at sampling temperatures under different loading conditions and ambient temperatures, in the hopes of identifying a pattern.

In the second traditional method, the average water content in an electrical transformer can be estimated by measuring the dielectric dissipation factor, between the terminals, at a range of frequencies. However, this simply results an average measure of water content for the entire electrical transformer. It cannot estimate the water content at specific locations within the electrical transformer.

The third traditional method measures the dew point. However, this is very labor intensive. It requires draining the insulating liquid from the electrical transformer, pulling a vacuum around the insulation, filling the vacuum with dry air, and waiting until some equilibrium is reached between the surface of the insulation and the air. The variation of the water content in the air, measured through the variation in its dew point, provides a good estimate of the average water content at the surface of the insulation. But again, this method cannot estimate the water content at specific locations within the electrical transformer, such as the hottest spot (i.e., hot-spot) of the electrical transformer during operation.

In contrast to these traditional methods, disclosed embodiments implement a thermochemical digital twin of the electrical transformer. This thermochemical digital twin represents a mathematical model of the conditions experienced by the real physical electrical transformer. Starting with the same conditions and undergoing the same loading and temperatures, the parameters estimated by the digital twin correspond accurately to those of the real electrical transformer.

Furthermore, no system is completely sealed, and chemical reactions, which generate and/or consume water, may occur inside the real electrical transformer. Thus, in an embodiment, the digital twin may utilize artificial intelligence (AI) to continuously correct the total water content inside the electrical transformer, based on measured values of water in the insulating liquid, as output by a moisture sensor in the monitoring system of the electrical transformer. For example, the water content, estimated by the digital twin, may be continually or continuously compared with the water content derived from the moisture sensor, and deviations between the two values of water content may be used to correct the total water content in the electrical transformer that is estimated by the digital twin. This keeps the calculations in the digital twin meaningful and technically sound.

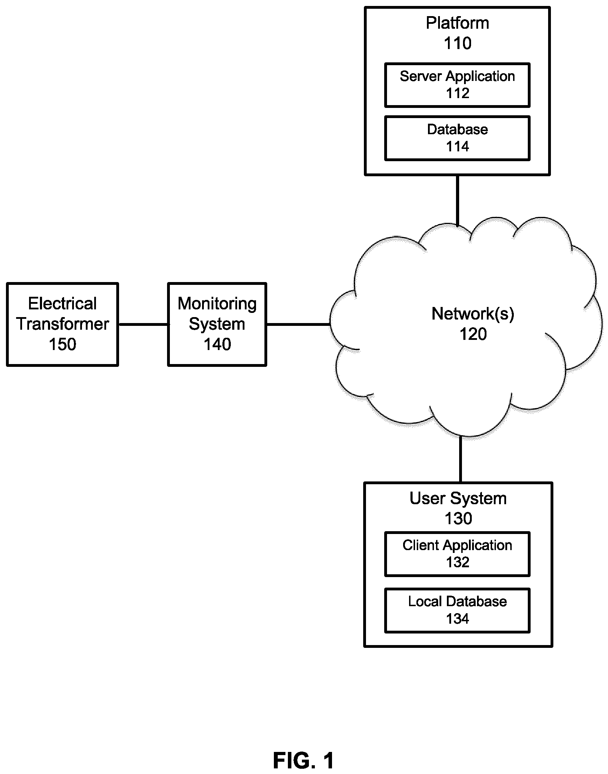

FIG. 1 illustrates an example infrastructure in which one or more of the disclosed processes may be implemented, according to an embodiment. The infrastructure may comprise a platform 110 (e.g., one or more servers) which hosts and/or executes one or more of the various processes, methods, functions, and/or software modules described herein, including, for example, the thermochemical digital twin. Platform 110 may comprise dedicated servers, or may instead be implemented in a computing cloud, in which the resources of one or more servers are dynamically and elastically allocated to multiple tenants based on demand. In either case, the servers may be collocated and/or geographically distributed. Platform 110 may execute a server application 112, which may utilize a database 114.

Platform 110 may be communicatively connected to one or more user systems 130 via one or more networks 120. Platform 110 may also be communicatively connected to one or more monitoring systems 140 via one or more networks 120. Each monitoring system 140 may monitor one or more parameters of one or more electrical transformers 150.

Network(s) 120 may comprise the Internet, and platform 110 may communicate with user system(s) 130 and/or monitoring system(s) 140 through the Internet using standard transmission protocols, such as HyperText Transfer Protocol (HTTP), HTTP Secure (HTTPS), File Transfer Protocol (FTP), FTP Secure (FTPS), Secure Shell FTP (SFTP), and the like, as well as proprietary protocols. While platform 110 is illustrated as being connected to various systems through a single set of network(s) 120, it should be understood that platform 110 may be connected to the various systems via different sets of one or more networks. For example, platform 110 may be connected to a subset of user systems 130 and/or monitoring systems 140 via the Internet, but may be connected to one or more other user systems 130 and/or monitoring systems 140 via an intranet. Furthermore, while only a single platform 110, user system 130, and monitoring system 140 is illustrated, it should be understood that the infrastructure may comprise any number of platforms 110, user systems 130, and monitoring systems 140.

A user system 130 may comprise any type of computing device capable of wired and/or wireless communication, including without limitation, desktop computers, laptop computers, tablet computers, smart phones or other mobile phones, servers, game consoles, televisions, set-top boxes, electronic kiosks, and/or the like. However, it is generally contemplated that user system 130 would comprise the workstation or personal computing device of a user with responsibility for operating and/or maintaining an electrical transformer 150. Each user system 130 may execute a client application 132, which may utilize a local database 134.

A monitoring system(s) 140 may comprise any type of device capable of wired and/or wireless communication. In the simplest form, monitoring system 140 may be a sensor that is configured to measure a parameter of electrical transformer 150 and output a signal, representing the value of the measured parameter, to platform 110. In a more complex form, monitoring system 140 could comprise a computing device that receives signals output by one or more sensors configured to measure one or more parameters of electrical transformer 150. In this case, monitoring system 140 may relay raw data, represented by the output signal(s) from the sensor(s) to platform 110, or preprocess the raw data and send the preprocessed data to platform 110. In any case, the sensor(s) may comprise a moisture sensor that measures a water content of the insulating liquid inside electrical transformer 150.

Platform 110 may comprise web servers which host one or more websites and/or web services. In embodiments in which a website is provided, the website may comprise a graphical user interface, including, for example, one or more screens (e.g., webpages) generated in HyperText Markup Language (HTML) or other language. Platform 110 may transmit or serve one or more screens of the graphical user interface in response to requests from user system(s) 130. In some embodiments, these screens may be served in the form of a wizard, in which case two or more screens may be served in a sequential manner, and one or more of the sequential screens may depend on an interaction of the user or user system 130 with one or more preceding screens. The requests to platform 110 and the responses from platform 110, including the screens of the graphical user interface, may both be communicated through network(s) 120, which may include the Internet, using standard communication protocols (e.g., HTTP, HTTPS, etc.). These screens (e.g., webpages) may comprise a combination of content and elements, such as text, images, videos, animations, references (e.g., hyperlinks), frames, inputs (e.g., textboxes, text areas, checkboxes, radio buttons, drop-down menus, buttons, forms, etc.), scripts (e.g., JavaScript), and the like, including elements comprising or derived from data stored in database 114. It should be understood that platform 110 may also respond to other requests from user system(s) 130 (e.g., unrelated to the graphical user interface).

Platform 110 may comprise, be communicatively coupled with, or otherwise have access to database 114. For example, platform 110 may comprise a database server that manages a database 114. Server application 112 executing on platform 110 and/or client application 132 executing on user system 130 may submit data (e.g., user data, form data, etc.) to be stored in database 114, and/or request access to data stored in database 114. Any suitable database may be utilized, including without limitation MySQL^{™}, Oracle^{™}, IBM^{™}, Microsoft SQL^{™}, Access^{™}, PostgreSQL^{™}, MongoDB^{™}, and the like, including cloud-based databases and proprietary databases. Data may be sent to platform 110, for instance, using the well-known POST request supported by HTTP, via FTP, and/or the like. This data, as well as other requests, may be handled, for example, by server-side web technology, such as a servlet or other software module (e.g., comprised in server application 112), executed by platform 110.

In embodiments in which a web service is provided, platform 110 may receive requests from user system(s) 130 and/or other external system(s) (e.g., which may themselves be servers), and provide responses in eXtensible Markup Language (XML), JavaScript Object Notation (JSON), and/or any other suitable or desired format. In such embodiments, platform 110 may provide an application programming interface (API) which defines the manner in which user system(s) 130 and/or other external system(s) may interact with the web service. Thus, user system(s) 130 and/or other external system(s) 140, can define their own user interfaces, and rely on the web service to implement or otherwise provide the backend processes, methods, functionality, storage, and/or the like, described herein. For example, in such an embodiment, a client application 132, executing on one or more user system(s) 130, may interact with a server application 112 executing on platform 110 to execute one or more or a portion of one or more of the various functions, processes, methods, and/or software modules described herein.

Client application 132 may be "thin," in which case processing is primarily carried out server-side by server application 112 on platform 110. A basic example of a thin client application 132 is a browser application, which simply requests, receives, and renders webpages at user system(s) 130, while server application 112 on platform 110 is responsible for generating the webpages and managing database functions. Alternatively, the client application may be "thick," in which case processing is primarily carried out client-side by user system(s) 130. It should be understood that client application 132 may perform an amount of processing, relative to server application 112 on platform 110, at any point along this spectrum between "thin" and "thick," depending on the design goals of the particular implementation. In any case, the software described herein, which may wholly reside on either platform 110 (e.g., in which case server application 112 performs all processing) or user system(s) 130 (e.g., in which case client application 132 performs all processing) or be distributed between platform 110 and user system(s) 130 (e.g., in which case server application 112 and client application 132 both perform processing), can comprise one or more executable software modules comprising instructions that implement one or more of the processes, methods, or functions described herein.

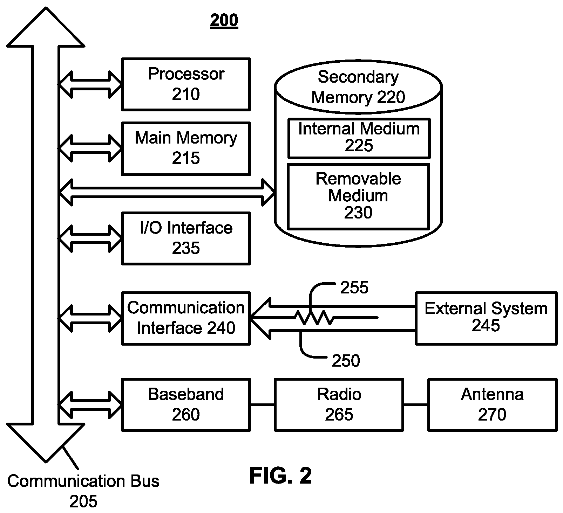

FIG. 2 illustrates an example wired or wireless system 200 that may be used in connection with various embodiments described herein. For example, system 200 may be used in conjunction with one or more of the processes, methods, or functions (e.g., to store and/or execute the software) described herein, and may represent components of platform 110, user system 130, monitoring system 140, and/or other processing devices described herein. System 200 can be any processor-enabled device (e.g., server, personal computer, etc.) that is capable of wired or wireless data communication. Other processing systems and/or architectures may also be used, as will be clear to those skilled in the art.

System 200 may comprise one or more processors 210. Processor(s) 210 may comprise a central processing unit (CPU) or main processor. Additional processors may be provided, such as a graphics processing unit (GPU), an auxiliary processor to manage input/output, an auxiliary processor to perform floating-point mathematical operations, a special-purpose microprocessor having an architecture suitable for fast execution of signalprocessing algorithms (e.g., digital-signal processor), a subordinate processor (e.g., back-end processor), an additional microprocessor or controller for dual or multiple processor systems, and/or a coprocessor. Such auxiliary processors may be discrete processors or may be integrated with the main processor. Examples of processors 210 which may be used with system 200 include, without limitation, any of the processors (e.g., Pentium^{™}, Core 17^{™}, Core i9^{™}, Xeon^{™}, etc.) available from Intel Corporation of Santa Clara, California, any of the processors available from Advanced Micro Devices, Incorporated (AMD) of Santa Clara, California, any of the processors (e.g., A series, M series, etc.) available from Apple Inc. of Cupertino, any of the processors (e.g., Exynos^{™}) available from Samsung Electronics Co., Ltd., of Seoul, South Korea, any of the processors available from NXP Semiconductors N.V. of Eindhoven, Netherlands, and/or the like.

Processor(s) 210 may be connected to a communication bus 205. Communication bus 205 may include a data channel for facilitating information transfer between storage and other peripheral components of system 200. Furthermore, communication bus 205 may provide a set of signals used for communication with processor 210, including a data bus, address bus, and/or control bus (not shown). Communication bus 205 may comprise any standard or non-standard bus architecture such as, for example, bus architectures compliant with industry standard architecture (ISA), extended industry standard architecture (EISA), Micro Channel Architecture (MCA), peripheral component interconnect (PCI) local bus, standards promulgated by the Institute of Electrical and Electronics Engineers (IEEE) including IEEE 488 general-purpose interface bus (GPIB), IEEE 696/S-100, and/or the like.

System 200 may comprise main memory 215. Main memory 215 provides storage of instructions and data for programs executing on processor 210, such as any of the software described herein. It should be understood that programs stored in the memory and executed by processor 210 may be written and/or compiled according to any suitable language, including without limitation C/C++, Java, JavaScript, Perl, Python, Visual Basic, .NET, and the like. Main memory 215 is typically semiconductor-based memory such as dynamic random access memory (DRAM) and/or static random access memory (SRAM). Other semiconductor-based memory types include, for example, synchronous dynamic random access memory (SDRAM), Rambus dynamic random access memory (RDRAM), ferroelectric random access memory (FRAM), and the like, including read only memory (ROM).

System 200 may comprise secondary memory 220. Secondary memory 220 is a non-transitory computer-readable medium having computer-executable code and/or other data (e.g., any of the software disclosed herein) stored thereon. As used herein, the term "computer-readable medium" refers to any non-transitory computer-readable storage media used to provide computer-executable code and/or other data to or within system 200. The computer software stored on secondary memory 220 is read into main memory 215 for execution by processor 210. Secondary memory 220 may include, for example, semiconductor-based memory, such as programmable read-only memory (PROM), erasable programmable read-only memory (EPROM), electrically erasable read-only memory (EEPROM), and flash memory (block-oriented memory similar to EEPROM).

Secondary memory 220 may include an internal medium 225 and/or a removable medium 230. Removable medium 230 is read from and/or written to in any well-known manner. Removable storage medium 230 may be, for example, a magnetic tape drive, a compact disc (CD) drive, a digital versatile disc (DVD) drive, other optical drive, a flash memory drive, and/or the like.

System 200 may comprise an input/output (I/O) interface 235. I/O interface 235 provides an interface between one or more components of system 200 and one or more input and/or output devices. Example input devices include, without limitation, sensors, keyboards, touch screens or other touch-sensitive devices, cameras, biometric sensing devices, computer mice, trackballs, pen-based pointing devices, and/or the like. Examples of output devices include, without limitation, other processing systems, cathode ray tubes (CRTs), plasma displays, light-emitting diode (LED) displays, liquid crystal displays (LCDs), printers, vacuum fluorescent displays (VFDs), surface-conduction electron-emitter displays (SEDs), field emission displays (FEDs), and/or the like. In some cases, an input and output device may be combined, such as in the case of a touch panel display (e.g., in a smartphone, tablet computer, or other mobile device).

System 200 may comprise a communication interface 240. Communication interface 240 allows data to be transferred between system 200 and external devices (e.g. printers), networks, or other information sources. For example, computer-executable code and/or other data may be transferred to system 200 from a network server (e.g., platform 110) via communication interface 240. Examples of communication interface 240 include a built-in network adapter, network interface card (NIC), Personal Computer Memory Card International Association (PCMCIA) network card, card bus network adapter, wireless network adapter, Universal Serial Bus (USB) network adapter, modem, a wireless data card, a communications port, an infrared interface, an IEEE 1394 fire-wire, and any other device capable of interfacing system 200 with a network (e.g., network(s) 120) or another computing device. Communication interface 240 preferably implements industry-promulgated protocol standards, such as Ethernet IEEE 802 standards, Fiber Channel, digital subscriber line (DSL), asynchronous digital subscriber line (ADSL), frame relay, asynchronous transfer mode (ATM), integrated digital services network (ISDN), personal communications services (PCS), transmission control protocol/Internet protocol (TCP/IP), serial line Internet protocol/point to point protocol (SLIP/PPP), and so on, but may also implement customized or non-standard interface protocols as well.

Data transferred via communication interface 240 is generally in the form of electrical communication signals 255. These signals 255 may be provided to communication interface 240 via a communication channel 250 between communication interface 240 and an external system 245 (e.g., which may correspond to a sensor of electrical transformer 150, an external computer-readable medium, and/or the like). In an embodiment, communication channel 250 may be a wired or wireless network (e.g., network(s) 120), or any variety of other communication links. Communication channel 250 carries signals 255 and can be implemented using a variety of wired or wireless communication means including wire or cable, fiber optics, conventional phone line, cellular phone link, wireless data communication link, radio frequency ("RF") link, or infrared link, just to name a few.

Computer-executable code is stored in main memory 215 and/or secondary memory 220. Computer-executable code can also be received from an external system 245 via communication interface 240 and stored in main memory 215 and/or secondary memory 220. Such computer-executable code, when executed, enables system 200 to perform the various functions of the disclosed embodiments.

In an embodiment that is implemented using software, the software may be stored on a computer-readable medium and initially loaded into system 200 by way of removable medium 230, I/O interface 235, or communication interface 240. In such an embodiment, the software is loaded into system 200 in the form of electrical communication signals 255. The software, when executed by processor 210, preferably causes processor 210 to perform one or more of the processes and functions described elsewhere herein.

System 200 may comprise wireless communication components that facilitate wireless communication over a voice network and/or a data network (e.g., in the case of user system 130). The wireless communication components comprise an antenna system 270, a radio system 265, and a baseband system 260. In system 200, radio frequency (RF) signals are transmitted and received over the air by antenna system 270 under the management of radio system 265.

In an embodiment, antenna system 270 may comprise one or more antennae and one or more multiplexors (not shown) that perform a switching function to provide antenna system 270 with transmit and receive signal paths. In the receive path, received RF signals can be coupled from a multiplexor to a low noise amplifier (not shown) that amplifies the received RF signal and sends the amplified signal to radio system 265.

In an alternative embodiment, radio system 265 may comprise one or more radios that are configured to communicate over various frequencies. In an embodiment, radio system 265 may combine a demodulator (not shown) and modulator (not shown) in one integrated circuit (IC). The demodulator and modulator can also be separate components. In the incoming path, the demodulator strips away the RF carrier signal leaving a baseband receive audio signal, which is sent from radio system 265 to baseband system 260.

Baseband system 260 is communicatively coupled with processor(s) 210, which have access to memory 215 and 220. Thus, software can be received from baseband processor 260 and stored in main memory 210 or in secondary memory 220, or executed upon receipt. Such software, when executed, can enable system 200 to perform the various functions of the disclosed embodiments.

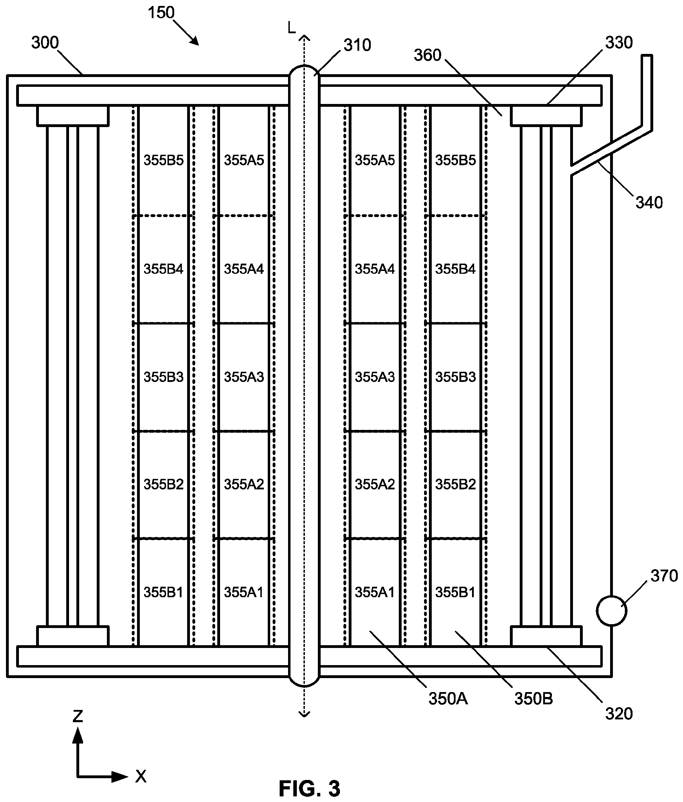

FIG. 3 illustrates a schematic representation of a discretized electrical transformer 150, according to an embodiment. It should be understood that the actual structure and dimensions of electrical transformer 150 are not limiting upon any embodiment. Rather, disclosed embodiments may be utilized with or adapted to any type, size, or model of electrical transformer 150.

Electrical transformer 150 may comprise a housing 300 that encloses one or more windings around a core 310. The winding(s) may comprise one or more lower stacks 320, one or more upper stacks 330, one or more leads 340, and one or more coils 350. In the illustrated embodiment, electrical transformer 150 has two coils 350A and 350B. However, it should be understood that electrical transformer 150 may comprise any number of coils 350, including one coil 350 or three or more coils 350. Lower stack(s) 320, upper stack(s) 330, and coil(s) 350 may each be annular and generally symmetric around core 310, and concentric around a longitudinal Z-axis L of core 310. Each lower stack 320 represents a first assembling element at a bottom end of the winding(s) along longitudinal axis L, and each upper stack 330 represents a second assembling element at an opposing top end of the winding(s) along longitudinal axis L. Lead 340 provides an electrical connection between the windings and a terminal of electrical transformer 150. Lead(s) 340 and coil(s) 350 may be formed from any suitable conductive material, such as copper, brass, bronze, aluminum, steel, and/or the like. Lower stack(s) 320, upper stack(s) 330, lead(s) 340, and coil(s) 350 may also comprise insulation, which may be formed from a cellulose-based material and is commonly referred to as "paper."

Housing 300 is filled with an insulating liquid 360, which surrounds the inner components of electrical transformer 150, including core 310, lower stack(s) 320, upper stack(s) 330, lead(s) 340, and coil(s) 350. The insulating liquid may comprise or consist of mineral oil, liquid ester, or another substance that is suitable for insulating the inner components of electrical transformer 150. Regardless of the particular substance, insulating liquid has a water content that will vary over time and operating conditions (e.g., loading and temperature).

The interior of electrical transformer 150 may be logically divided into a plurality of sections along one or more axes. For example, each coil 350 is divided into a plurality of sections 355 along the Z-axis. In particular, coil 350A is divided into sections 355A1, 355A2, 355A3, 355A4, and 355A5 from a bottom end to a top end of the windings along the Z-axis. Similarly, coil 350B is divided into sections 355B1, 355B2, 355B3, 355B4, and 355B5 from the bottom end to the top end of the windings along the Z-axis. It should be understood that each coil 350 may be divided into any number of sections, including fewer than five sections (and potentially, only a single section) or more than five sections. The plurality of sections for the entire electrical transformer 150 may include each of the coil sections 355, as well as at least one section representing lower stack(s) 320, at least one section representing upper stack(s) 330, and/or at least one section representing lead 340. Thus, the insulated inner components of electrical transformer 150 can be logically divided along both the Z-axis and the radial X-axis. It should be understood that the sections representing lower stack(s) 320, upper stack(s) 330, and coil(s) 350 may be annular around longitudinal axis L, or may each be further divided into annulus sectors around longitudinal axis L. Each of the plurality of sections may be associated with a mass and thickness of the insulation in the section, based on proportional values of the total mass and thickness of the insulation in the entire electrical transformer 150.

Electrical transformer 150 may also comprise one or more sensor(s) 370. Sensor(s) 370 may comprise a moisture sensor that senses the water content in insulating liquid 360. Moisture sensor 370 may output a signal indicating the water content in insulating liquid 360 to monitoring system 140 or directly to platform 110, for use as an input to the thermochemical digital twin described herein. In an embodiment, the water content in insulating liquid 360 is assumed to be uniform inside electrical transformer 150.

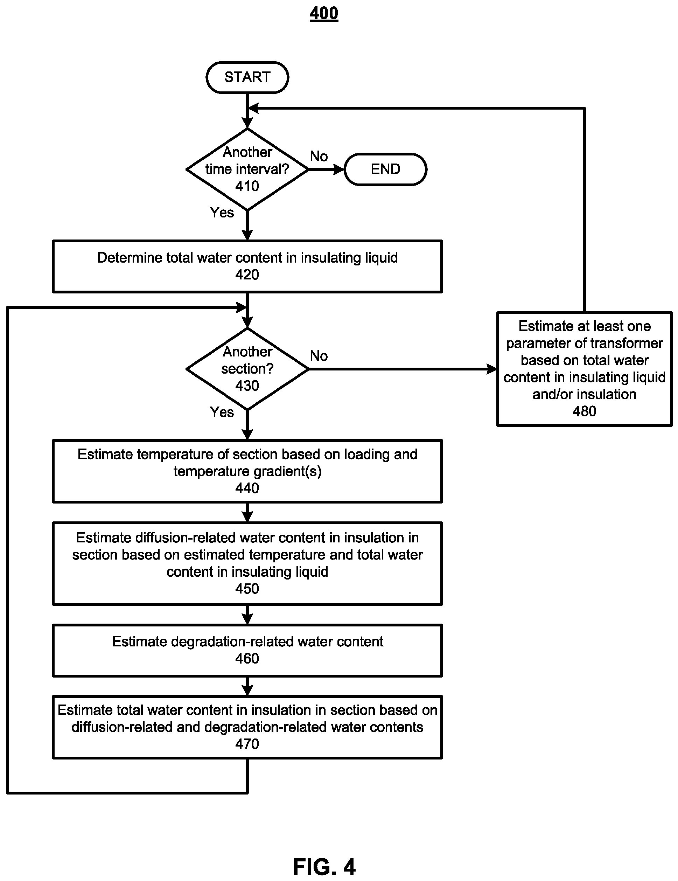

FIG. 4 illustrates a process 400 for modeling thermochemical degradation in a digital twin of an electrical transformer 150, according to an embodiment. While process 400 is illustrated with a certain arrangement and ordering of subprocesses, process 400 may be implemented with fewer, more, or different subprocesses and a different arrangement and/or ordering of subprocesses. In addition, it should be understood that any subprocess, which does not depend on the completion of another subprocess, may be executed before, after, or in parallel with that other independent subprocess, even if the subprocesses are described or illustrated in a particular order.

Process 400 may be embodied in one or more software modules that are executed by one or more hardware processors (e.g., processor 210) on a system 200, for example, as a software application (e.g., server application 112, client application 132, and/or a distributed application comprising both server application 112 and client application 132), which may be executed wholly by processor(s) of platform 110, wholly by processor(s) of user system(s) 130, or may be distributed across platform 110 and user system(s) 130, such that some modules of the software application are executed by platform 110 and other modules of the software application are executed by user system(s) 130. Process 400 may be implemented as instructions represented in source code, object code, and/or machine code. These instructions may be executed directly by hardware processor(s) 210, or alternatively, may be executed by a virtual machine operating between the object code and hardware processor(s) 210. Alternatively, process 400 may be implemented as a hardware component (e.g., general-purpose processor, integrated circuit (IC), application-specific integrated circuit (ASIC), digital signal processor (DSP), field-programmable gate array (FPGA) or other programmable logic device, discrete gate or transistor logic, etc.), combination of hardware components, or combination of hardware and software components.

Process 400 may iterate for one or a plurality of time intervals. When another time interval remains (i.e., "Yes" in subprocess 410), process 400 may perform an iteration of an outer loop comprising subprocesses 420-480. Otherwise, when no time intervals remain (i.e., "No" in subprocess 410), process 400 may end. In an embodiment, process 400 may execute an iteration of the outer loop at a plurality of fixed time intervals (e.g., a number of milliseconds, seconds, minutes, hours, days, etc.) for as long as the electrical transformer 150, which is being modeled by the digital twin, is operational. In other words, the determination may be "Yes" in subprocess 410 for as long as electrical transformer 150 is operational and being monitored. Thus, for each electrical transformer 150 being monitored and in operation, the digital twin of that electrical transformer 150 may execute process 400 in real time based on input data acquired for the current time interval. As used herein, the term "real time" or "real-time" should be understood to include an event (e.g., iteration of the outer loop in process 400) that is delayed from another event (e.g., acquisition of the data used as input to the iteration of the outer loop in process 400) by ordinary latencies in computer processing, network communications, data storage and retrieval, and/or the like, as well as events that occur simultaneously with each other. The determination may be "No" in subprocess 410 as soon as electrical transformer 150 becomes non-operational or monitoring of electrical transformer 150 is terminated for some reason.

In subprocess 420, a total water content in insulating liquid 360 of electrical transformer 150 is determined. In an embodiment, subprocess 420 comprises deriving the total water content in insulating liquid 360 from the output of at least one sensor 370, which may comprise a moisture sensor in electrical transformer 150. In an alternative embodiment, the total water content in insulating liquid 360 may be determined manually, using an estimation algorithm, retrieving a value from a lookup table based on one or more other parameter values, or in any other suitable manner.

As discussed above, in an embodiment, electrical transformer 150 is logically divided into a plurality of sections along at least one axis of the winding(s) in electrical transformer 150. When another one of the plurality of sections remains to be considered (i.e., "Yes" in subprocess 430), process 400 may perform an iteration of an inner loop comprising subprocesses 440-470 for that section. Otherwise, when no more of the plurality of sections remain to be considered (i.e., "No" in subprocess 430), process 400 may proceed to subprocess 480. It should be understood that each and every one of the plurality of sections will be considered exactly once during each iteration of the outer loop for a given time interval. While it is generally contemplated that there will be a plurality of sections, in an alternative embodiment, there may be only a single section (e.g., encompassing the entire insulation of electrical transformer 150). In this case, only one iteration of the inner loop would be performed for each iteration of the outer loop.

It should be understood that each iteration of the inner loop, comprising subprocesses 440-470, may not necessarily be performed individually and successively for each of section. While it could be the case that iterations of the inner loop are performed individually for each section, either in serial or in parallel, this is not a requirement of any embodiment. Rather, in an alternative embodiment, each of subprocesses 440-470 may be performed for all of the sections at once in a block of combined calculations.

Subprocess 440 represents the thermal model of the thermochemical digital twin. In subprocess 440, a temperature of the section, currently being considered in the inner loop, is estimated based on a loading of electrical transformer 150 and a temperature gradient along at least one axis. To account for the transient conditions in electrical transformer 150, the temperature of the section may be calculated for each of a plurality of time instants spanning the current time interval under consideration. Alternatively, the time interval may consist of only a single time instant.

For each time interval, process 400 may comprise determining an average temperature of insulating liquid 360 for each time instant in the time interval. The average temperature of insulating liquid 360 may be calculated using the equations for the transient calculation of temperature based on loading conditions from IEEE C57.91-2011, clause 7. In particular, these equations are: $Δ {θ}_{TO} = \left(Δ{θ}_{TO , U}-Δ{θ}_{TO , i}\right) ⋅ \left(1-{e}^{- \frac{t}{{τ}_{TO}}}\right) + Δ {θ}_{TO , i}$ wherein Δ*θ_{TO}* is the average temperature rise of insulating liquid 360 over the ambient temperature, Δ*θ_{TO,U}* is the ultimate temperature rise of insulating liquid 360 over the ambient temperature for the current loading of electrical transformer 150, Δ*θ_{TO,i}* is the initial temperature rise of insulating liquid 360 over the ambient temperature for the previous loading, e is Euler's number, *t* is the time instant, and τ*_{TO}* is the time constant of insulating liquid 360 for any loading and any specific temperature differential between the ultimate temperature rise Δ*θ_{TO,U}* and the initial temperature rise Δ*θ_{TO,i}.* $Δ {θ}_{TO , i} = Δ {θ}_{TO , R} {\left[\frac{\left({K}_{i}^{2}R+1\right)}{\left(R+1\right)}\right]}^{n}$ wherein Δ*θ_{TO,R}* is the temperature rise of insulating liquid 360 over the ambient temperature at the rated load on the tap position, *Kᵢ* is the ratio of the previous loading to the rated load per unit, n is an empirically derived exponent used to calculate the variation of the temperature rise of insulating liquid 360 with load changes (e.g., defined in a table), and R is the ratio of load loss at the rated load to no load loss on the tap position. ${τ}_{TO , R} = \frac{C Δ {θ}_{TO , R}}{{P}_{T , R}}$ wherein *τ_{TO,R}* is the time constant for the rated load beginning with the initial top-oil temperature rise of 0°C, *C* is the initial temperature rise of insulating liquid 360 over the ambient temperature for the previous loading, and *P_{T,R}* is the total loss at the rated load. ${τ}_{TO} = {τ}_{TO , R} \frac{\left(\frac{Δ {θ}_{TO , U}}{Δ {θ}_{TO , R}}\right) - \left(\frac{Δ {θ}_{TO , i}}{Δ {θ}_{TO , R}}\right)}{{\left(\frac{Δ {θ}_{TO , U}}{Δ {θ}_{TO , R}}\right)}^{\frac{1}{n}} - {\left(\frac{Δ {θ}_{TO , i}}{Δ {θ}_{TO , R}}\right)}^{\frac{1}{n}}}$

The above equations can be applied to values of loading and ambient temperature for each time instant to calculate the average temperature of insulating liquid 360 at each time instant. It should be understood that this is one example of how to calculate the average temperature of insulating liquid 360. In an alternative embodiment, the average temperature of insulating liquid 360 may be determined in any other suitable manner.

In addition to the average temperature of insulating liquid 360, the vertical temperature gradient for the loading may be calculated for each time instant. The vertical temperature gradient represents the difference in the temperature of insulating liquid 360 between the bottom end and top end of the winding(s) along the Z-axis. The vertical temperature gradient is directly proportional to the current loading.

Similarly, an axial temperature gradient for the loading may be calculated for each time instant. The axial temperature gradient represents the difference in the temperature of insulating liquid 360 from the center to the outer circumference of housing 300 along the radial X-axis. The axial temperature gradient may also be directly proportional to the current loading.

In an embodiment, a winding-to-oil temperature gradient may also be calculated or otherwise determined. The winding-to-oil temperature gradient accounts for the temperature between the conductor and insulating liquid 360, which may vary across sections along the Z-axis and/or radial X-axis.

The localized temperature of each section of the winding(s) may be calculated based on the average temperature of insulating liquid 360, the vertical temperature gradient, the axial temperature gradient, and/or the winding-to-oil temperature gradient. For example, subprocess 440 may comprise estimating the temperature of each section by, for each time instant, calculating a localized temperature of lower stack(s) 320, based on the average temperature of insulating liquid 360 and the axial temperature gradient calculated for the time instant, calculating a localized temperature of upper stack(s) 330 based on the average temperature of the insulating liquid and the axial temperature gradient calculated for the time instant, and calculating a localized temperature for each coil section 355, based on the temperature of lower stack(s) 320 or upper stack(s) 330, the loading, and the radial and axial temperature gradients.

As an example, the localized temperature of lower stack(s) 320 may be calculated as or otherwise based on a difference between the average temperature of insulating liquid 360 and half of the vertical temperature gradient for the current loading. The value of this difference represents the bottom temperature of insulating liquid 360 at the bottom end of the winding(s). In an embodiment in which there is a plurality of lower stacks 320 along the radial X-axis, the localized temperature of each lower stack 320 may be further determined based on the axial temperature gradient.

As an example, the localized temperature of upper stack(s) 330 may be calculated as or otherwise based on the sum of the average temperature of insulating liquid 360 and half of the vertical temperature gradient for the loading. The value of this sum represents the top temperature of insulating liquid 360 at the top end of the winding(s). In an embodiment in which there is a plurality of upper stacks 330 along the radial X-axis, the localized temperature of each upper stack 330 may be further determined based on the axial temperature gradient.

As an example, the localized temperature of each coil section 355 may be calculated as or otherwise based on the sum of the bottom temperature (e.g., the localized temperature of lower stack(s) 320), a percentage of the vertical temperature gradient, and half of the winding-to-oil temperature gradient. Alternatively, the localized temperature of each coil section 355 could be calculated as or otherwise based on the sum of the top temperature (e.g., the localized temperature of upper stack(s) 330) and half of the winding-to-oil temperature gradient, minus a percentage of the vertical temperature gradient. In either case, the percentage of the vertical temperature gradient that is used for a particular coil section 355 may be proportional to the height range of that coil section 355 along the Z-axis of electrical transformer 150. For instance, using coil 350A as an example, if there are five coil sections 355A1-355A5, the percentage of the vertical temperature gradient for the bottommost and first coil section 355A1 may be 10%, the percentage of the vertical temperature gradient for second coil section 355A2 may be 30%, the percentage of the vertical temperature gradient for third coil section 355A3 may be 50%, the percentage of the vertical temperature gradient for fourth coil section 355A4 may be 70%, and the percentage of the vertical temperature gradient for the topmost and fifth coil section 355A5 may be 100%. More generally, the percentage of the vertical temperature gradient for each coil section 355 may be determined as the middle of the height range represented by the coil section 355 (e.g., 30% for third coil section 355A3, because third coil section 355A3 represents the range of 20-40% in the total height of coil 350A). In an embodiment in which there is a plurality of coils 350 along the radial X-axis, the localized temperature of each coil section 355 may be further determined based on the axial temperature gradient. For example, the localized temperature of each coil section 355A1-355A5 of coil 350A may be determined using a different percentage of the axial temperature gradient than each coil section 355B1-355B1 of coil 350B.

In an embodiment, in each coil 350, the localized temperature of the one coil section 355 that is closest to the top end of electrical transformer 150 may be further based on a hot-spot factor or heat-dissipation factor associated with electrical transformer 150. In other words, the localized temperature of the topmost coil section 355 of each coil 350 may be adjusted based on this factor, to represent that the topmost coil section 355 is the hottest region of each coil 350. The hot-spot factor or heat-dissipation factor may be calculated or otherwise determined (e.g., retrieved as a predefined constant) in any suitable manner.

Whereas subprocess 440 represents the thermal model, subprocesses 450-470 represent the chemical model of the thermochemical digital twin. In subprocess 450, a diffusion-related water content in the insulation of the winding(s) in the section, currently being considered in the inner loop, resulting from diffusion, is estimated based on the localized temperature for the section, determined in subprocess 440, and the total water content in insulating liquid 360.

The loading conditions of an electrical transformer 150 typically do not remain steady for a sufficient duration to reach an equilibrium condition. Thus, in an embodiment, the diffusion process is modeled for transient conditions. In particular, subprocess 450 may comprise, for each of the plurality of time instants spanning the time interval, calculating a water content diffused into the insulation based on a time constant for diffusion of water into the material of the insulation.

The diffusion process during transient conditions may be modeled as an exponential process that is governed by a time constant that is calculated based on the temperature, water content in insulating liquid 360, thickness of the insulation, and/or geometry of the insulation. For example, for each time instant *t*, the water content diffused into the insulation may be calculated in subprocess 450 as: ${W}_{diffusion} = \left({W}_{equilibrium}-{W}_{initial}\right) ⋅ \left(1-{e}^{- \frac{t}{{τ}_{p}}}\right) + {W}_{initial}$ wherein *W_{diffusion}* is the water content diffused into the insulation at time instant *t, W_{equilibrium}* is a water content in the insulation at the equilibrium condition, *Wᵢₙᵢₜᵢₐₗ* is a water content in the insulation at a time instant immediately preceding time instant *t* (i.e., at time instant *t -* 1), *e* is Euler's number, and *τₚ* is the time constant.

In an embodiment in which insulating liquid 360 is mineral oil and the insulation is cellulose-based, the value of *W_{equilibrium}* may be derived based on the balance of water depicted in the set of equilibrium curves in Oomen, "Moisture Equilibrium in Paper-Oil Systems," Proceedings of the IEEE Electrical Insulation Conference, Chicago, IL, pp. 162-166, October 1983. For example, the water content in the insulation, at the equilibrium condition, may be calculated as: ${W}_{equilibrium} = {Me}^{K ∗ T}$ $M = {M}_{slope} ⋅ {RS}_{oil} + {M}_{intercept}$ $K = {K}_{slope} ⋅ {RS}_{oil} + {K}_{intercept}$ ${RS}_{oil} = \frac{{W}_{oil}}{\frac{1670}{{10}^{T} + 273.15} + 7.42}$ wherein *W_{equilibrium}* is the water content in the insulation from diffusion at the equilibrium condition, *Wₒᵢₗ* is the water content in insulating liquid 360, *T* is the equilibrium temperature, *RSₒᵢₗ* is the relative saturation of water content in insulating liquid 360 (i.e., a ratio between water content and saturation content), and *Mₛₗₒₚₑ*, *M_{intercept}, Kₛₗₒₚₑ*, and *K_{intercept}* are coefficients defined by linear segments whose slope and intercept may be given for a given value of *RSₒᵢₗ* as follows:

| *RSₒᵢₗ* (%) | *Mₛₗₒₚₑ* | *M_{intercept}* | *Kₛₗₒₚₑ* | *K_{intercept}* |
|---|---|---|---|---|
| 0 | 48.055 | 0 | 0 | -0.01155245 |
| 5 | 14.411 | 1.6822 | 0.051383 | -0.014122 |
| 10 | 12.179 | 1.9055 | 0.0095265 | -0.0099359 |
| 20 | 12.358 | 1.8696 | 0.0019723 | -0.0084251 |
| 30 | 9.5961 | 2.6982 | 0.0059252 | -0.009611 |
| 40 | 11.168 | 2.0696 | 0.0034817 | -0.0086336 |
| 50 | 12.061 | 1.623 | 0.0041801 | -0.0089828 |
| 60 | 13.053 | 1.0276 | 0.0034595 | -0.0085504 |
| 70 | 14.065 | 0.3191 | 0.01389 | -0.015851 |
| 80 | 32.112 | -14.118 | 0.0095796 | -0.012403 |
| 90 | 83.494 | -60.362 | -0.011016 | 0.0061326 |
| 95 | 217.31 | -187.48 | -0.022849 | 0.017374 |
| 100 | 217.31 | -187.48 | -0.022849 | 0.017374 |

In an embodiment, the time constant *τₚ* may be calculated based on the disclosure of Du et al., "Moisture Equilibrium in Transformer Paper-Oil Systems," Dielectrics and Electrical Insulation Society (DEIS) Electrical Insulation Magazine, Featured Article, vol. 15, no. 1, Jan./Feb. 1999. For example, the time constant *τₚ* may be calculated as:
${τ}_{p} = \frac{{d}^{2}}{{π}^{2} D}$, when moisture ingress occurs through two surfaces of the insulation
${τ}_{p} = \frac{4 {d}^{2}}{{π}^{2} D}$, when the moisture ingress occurs through only one surface of the insulation wherein *d* is the thickness of the insulation material, and *D* is a coefficient. In an embodiment, coefficient D is calculated as: $D = {D}_{0} {e}^{0.5 C + {E}_{a} \left(\frac{1}{{T}_{0}}-\frac{1}{T}\right)}$ wherein *D*₀ = 1.34 × 10⁻¹³ m²/s, *C* is the moisture concentration in percentage by weight, *Eₐ* = 8074 K for an oil-impregnated system, *T*₀ = 298 K, and *T* is the temperature of the section, as determined in subprocess 440, in units of Kelvin.

In subprocess 460, a degradation-related water content, based on degradation of a material of the insulation of the winding(s) in the section, currently being considered in the inner loop, is estimated. If the insulation of the winding(s) is cellulose-based, the degradation-related water content may be estimated using the stochiometric relation of cellulose degradation. In particular, the degradation-related water content in the insulation may be estimated based on a water generation rate from scissions of cellulose chains in the cellulose-based material.

As discussed in Lundgaard et al., "Aging of Oil-Impregnated Paper in Power Transformers," IEEE Transactions on Power Delivery, vol. 19, no. 1, Jan. 2004 ("Lundgaard"), there is a net production of two molecules of water per glucose monomer. Considering that each scission in cellulose degradation leads to the reaction of two glucose monomers, there will be a net generation of four molecules of water per scission.

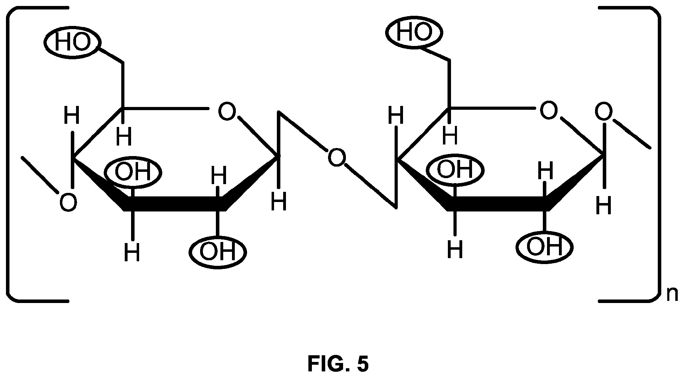

FIG. 5 illustrates a chemical representation of two monomers of glucose (C₆H₁₀O₅)ₙ, in which the hydroxyls that lead to the generation of water are circled. From the six circled hydroxyls, two will be consumed in the chain of reactions. A complete degradation of a mass of cellulose would require approximately eight to ten scissions of the cellulose chain.

Lundgaard depicted a relatively linear trend for the number of water molecules that are generated in successive scissions of cellulose degradation. However, the rate of generation should follow an exponential curve. Accordingly, in an embodiment, the water generation from cellulose degradation is modeled as having an exponential behavior. The decay of the viscometric degree of polymerization accounts for this exponential behavior, which justifies the adoption of a constant rate of water generation per unit of life consumed.

The scission of the cellulose molecule may occur at any point in the cellulose chain, which leads to the generation of four molecules of water and, on average, the halving of the molecular weight of the cellulose chain. Thus, the number of water molecules generated by successive scissions of the cellulose chain can be expressed by a geometric progression, starting with four and doubling for each new scission. The integration of this curve can then be divided by the molecular weight of the cellulose (e.g., which starts with the number of glucose monomers defined by the degree of polymerization), and multiplied by the molecular weight of water. In this case, the rate of water generation from cellulose degradation, for *k* successive decays of the degree of polymerization, may be modeled as: ${W}_{rate} = {\int }_{0}^{k} {2}^{x + 1} dx ⋅ \frac{{Mol}_{water}}{DP ⋅ {Mol}_{glucose}} ⋅ \frac{1}{UoL}$ wherein *Wᵣₐₜₑ* is the rate of water generation from cellulose degradation, *k* is the number of scissions of the cellulose chain (e.g., *k = 8), Mol_{water}* is the molar mass of water (e.g., *Mol_{water}* = 18.01528 grams per mole), *DP* is the degree of polymerization of the cellulose-based material of the insulation, defined as a number of monomer units (e.g., *DP =* 1200 monomers), *Mol_{glucose}* is the molar mass of a monomer of glucose (e.g., *Mol_{glucose} =* 162.1406 grams per mole), and UoL is a unit of life for electrical transformer 150. The IEEE defines the unit of life for an electrical transformer as UoL = 180,000 hours, based on results of the Lockie Test and a safety factor of five times. However, it should be understood that other values may be used for the unit of life UoL. In addition, it should be understood that *Wᵣₐₜₑ* will be defined as a water content in the units defined by *Mol_{water}* (e.g., grams) per unit of monomer mass defined by *DP* · *Mol_{glucose}* (e.g., grams) per the unit of time defined by UoL (e.g., hour). If the insulation has a viscometric degree of polymerization *DP =* 1200 and is subjected to *k* = 8 scissions (which will result in a low average *DP* value), and the unit of life UoL = 180,000 hours, the rate of water generation *Wᵣₐₜₑ*, as calculated above, is 68.4 milligrams of water per gram of insulation per hour. Notably, this model for the rate of water generation closely aligns with the empirical observations in Lundgaard.

It should be understood that the degradation-related water content for a time period, such as the current time interval, may be calculated by multiplying the rate of water generation *Wᵣₐₜₑ* by the length of the time period. For example, if the unit of time in *Wᵣₐₜₑ* is an hour, and the time interval is one minute, the degradation-related water content generated during the time interval may be calculated as *Wᵣₐₜₑ*/60.

In subprocess 470, a total water content within the insulation of the winding(s) in the section, currently being considered in the inner loop, is estimated based on the diffusion-related water content, estimated in subprocess 450, and the degradation-related water content, estimated in subprocess 460. For example, the total water content within the insulation of the winding(s) in the section may be estimated as the sum of the diffusion-related water content and the degradation-related water content.

In subprocess 480, at least one parameter of electrical transformer 150 may be estimated based on the total water content in insulating liquid 360, as determined in subprocess 420, and/or the total water content in the insulation of the winding(s), as determined in iteration(s) of subprocess 470. The at least one parameter may comprise the total water content in electrical transformer 150, a measure of aging of electrical transformer 150, a risk of dielectric failure, a likelihood of microbubbles on the surface of the insulation, a breakdown voltage, and/or the like.

In an embodiment of subprocess 480, the at least one parameter comprises the total water content in electrical transformer 150. In this case, the total water content in electrical transformer 150 may be estimated based on the total water content in insulating liquid 360 and the total water content within the insulation of the winding(s) in all of the plurality of sections. The total water content within the insulation of the winding(s) in all of the plurality of sections may be determined as the sum of the estimated total water content in the insulation of each section, as determined in each iteration of subprocess 470 in the inner loop, across all of the plurality of sections.

In this embodiment of subprocess 480, the total water content in electrical transformer may be estimated further based on an adjustment parameter that is determined using artificial intelligence (AI). Electrical transformer 150 may not be a perfectly sealed system and may involve complex chemical processes and reactions, which may result in hydrolysis and/or pyrolysis. The adjustment parameter acts as leakage factor that may account for ingress of water into electrical transformer 150, egress of water out of electrical transformer 150, and/or any chemical reactions, which generate and/or consume water, that occur inside real electrical transformer 150 and are not accounted for by the other components of the thermochemical digital twin discussed herein.

The artificial intelligence may comprise an AI model of the thermochemical digital twin of electrical transformer 150 that outputs an estimated value of the total water content in insulating liquid 360. In each time interval, subprocess 480 may comprise calculating a deviation between an actual value of the total water content in insulating liquid 360, derived from the output of sensor 370, and one or more estimated values of the total water content in insulating liquid 360 output by the AI model. Apart from changes in the water content in electrical transformer 150, the deviation may vary with the temperature of insulating liquid 360 and the rate of change in the load over time. Subprocess 480 may determine the adjustment parameter based on the calculated deviation, and utilize the adjustment parameter in the estimate of the total water content in electrical transformer 150. For example, the adjustment parameter, which may be positive or negative, may be summed with the total water content in electrical transformer 150, to thereby adjust the estimate of the total water content in electrical transformer 150. Thus, the AI model can track deviations from the measured value of water content in insulating liquid 360, and continually adjust an adjustment parameter as a correction to keep the thermochemical digital twin of electrical transformer 150 consistent with the actual, physical electrical transformer 150.

The AI model may comprise a Recurrent Neural Network (RNN) or other artificial neural network, which may utilize long short-term memory (LSTM). Alternatively, the AI model may comprise a K-Nearest Neighbor (KNN) algorithm. The AI model may be trained using supervised learning or unsupervised learning. In supervised learning, the AI model may be trained using a labeled dataset comprising one or more features (e.g., temperature) that are labeled with one or more target values (e.g., total water content in insulating liquid 360).

As an example, a KNN algorithm operates by finding the k-nearest neighbors to a set of feature(s), and then estimating the target value based on the average of the k-nearest neighbors. A historical set of measured features (e.g., the temperature and the water content in insulating liquid 360, as measured by sensor 370) for each of a plurality of past time intervals may be maintained, along with the corresponding estimated water content in insulating liquid 360 for each of the plurality of past time intervals. The historical set of measured and estimated features may be maintained as a sliding window (e.g., representing the most recent past forty-eight hours). The deviation may be calculated by finding the k-nearest neighbors to the current measured features (e.g., the current temperature and the current water content in insulating liquid 360, as measured by sensor 370), and calculating the average deviation between the measured water content, in insulating liquid 360, of those k-nearest neighbors and the corresponding estimates of water content in insulating liquid 360. This average deviation is what is used to determine the adjustment parameter (e.g., by converting the average deviation into the unit of water content). In addition, if the value of the average deviation exceeds a predefined tolerance value (e.g., 0.5%), the AI model may increase the number of k-nearest neighbors that are used, such that more nearest neighbors are used to calculate the average deviation, until the average deviation is minimized and the average deviation falls below the predefined tolerance value. By optimizing the number of k-nearest neighbors, this process will minimize the error between the calculated and measured values of water content in insulating liquid 360.

In an alternative or additional embodiment of subprocess 480, the at least one parameter comprises an overall measure of aging of electrical transformer 150. In this case, process 400 may further comprise, for each of the time interval(s), for each of the section(s) of the winding(s) in electrical transformer 150, calculating a measure of aging for the section based on the total water content in the insulation of the winding(s) in the section. The overall measure of aging may be determined based on these measures of aging for the section(s). For example, the overall measure of aging may be determined as the maximum of the measures of aging for the section(s).

In this embodiment of subprocess 480, the measure of aging for each section may be calculated as: $V = \frac{A}{{A}_{r}} ⋅ {e}^{\frac{1}{R} \times \left(\frac{{E}_{r}}{{θ}_{h , r} + 273}-\frac{E}{{θ}_{h} + 273}\right)}$ wherein *V* is the measure of aging, A is an interpolated aging parameter associated with the material of the insulation, *Aᵣ* is a rated aging parameter associated with the material of the insulation, *e* is Euler's number, *R* is the molar gas constant (e.g., *R =* 8.31446261815324 J K⁻¹ mol⁻¹), *Eᵣ* is the activation energy (e.g., Arrhenius activation energy) of the material of the insulation at rated conditions, E is an interpolated activation energy (e.g., Arrhenius activation energy) of the material of the insulation, *θ_{h,r}* is a hot-spot-to-top-oil temperature at a rated load, and *θₕ* is a hot-spot temperature. The values of the rated aging parameter *Aᵣ* and the rated activation energy *Eᵣ* may be predefined constants.

The value of the measure of aging *V* is an aging acceleration factor that represents how many equivalent units of life (e.g., hours) of electrical transformer 150 have been consumed. A value of *V* > 1 indicates that electrical transformer 150 is aging faster than it is expected to under rated conditions, and a value of *V* < 1 indicates that electrical transformer 150 is aging slower than it is expected to under rated conditions. A value of *V* = 1 indicates that electrical transformer 150 is aging at the exact expected pace for rated conditions.

The values of A and *E* may be determined from a lookup table, for example, by retrieving their values, based on the water content in the insulation, oxygen content, acidity in insulating liquid 360, and/or the like. An example of one possible lookup table is given in International Electrotechnical Commission (IEC) 60076-7 Annex A, Table A1, which is sampled below for thermally upgraded paper insulation:

| | | Paper Insulation Type | | | |
|---|---|---|---|---|---|
| | | Free from air 0.5% moisture | Free from air 1.5% moisture | Free from air 3.5% moisture | With air 0.5% moisture |
| Parameters | A (hr⁻¹) | 1.6 × 10⁴ | 3.0 × 10⁴ | 6.1 × 10⁴ | 3.2 × 10⁴ |
| | *E* (kJ/mol) | 86 | 86 | 86 | 82 |

Alternatively, A and *E* may be retrieved from a different lookup table, or determined in another suitable manner. If the lookup table does not include a value of a parameter, A and/or E, for a particular moisture (i.e., water content in the insulation), the value of the parameter may be interpolated from the pair of values of the parameters for the surrounding moistures that are included in the lookup table. For example, if the paper insulation is free from air and has 0.9% moisture, the value of A may be interpolated as a proportional value between 1.6 × 10⁴ for 0.5% moisture and 3.0 × 10⁴ for 1.5% moisture (e.g., *A* = 2.16 × 10⁴). Then, a ratio between the rated aging parameter *Aᵣ* and the interpolated aging parameter A may be calculated as $\frac{A}{{A}_{r}} ,$, representing an aging ratio. The value of activation energy *E* may be similarly interpolated. It should be understood that the water content in the insulation, used for the lookup, is the total water content within the insulation in the winding(s) in each section, as estimated in subprocess 470. Notably, the values of A and *E* are based on an oxygen content (i.e., free from air vs. with air) in the insulation, such that the measure of aging is based on an oxygen content in the insulation. Additionally or alternatively, the values of A and/or *E* and/or another parameter may be based on an acidity of insulating liquid 360, such that the measure of aging is based on the acidity of insulating liquid 360.

The value of ${e}^{\frac{1}{R} \times \left(\frac{{E}_{r}}{{θ}_{h , r} + 273}-\frac{E}{{θ}_{h} + 273}\right)}$ represents an exponentiation factor. The measure of aging is calculated based on the aging ratio and the exponentiation factor, for example, as a product of the aging ratio and the exponentiation factor. The values of hot-spot-to-top-oil temperature *θ_{h,r}* and hot-spot temperature *θₕ* may be predefined constants, may be calculated based on one or more other parameters, or may be determined in any other suitable manner.

In an alternative or additional embodiment of subprocess 480, the at least one parameter comprises a risk of dielectric failure in the insulation of the winding(s). This risk of dielectric failure may be estimated based on the total water content within the insulation, which may be estimated as described above. For example, the risk of dielectric failure may be determined as a function of water content in the insulation based on the relationship described in Balma et al., "The Effects of Long Term Operation and System Conditions on the Dielectric Capability and Insulation Coordination of Large Power Transformers," IEEE Transactions on Power Delivery, vol. 14, no. 3, July 1999 ("Balma").

In an alternative or additional embodiment of subprocess 480, the at least one parameter comprises a likelihood of microbubbles on the surface of the insulation of the winding(s). This likelihood of microbubbles may be estimated based on the total water content within the insulation, which may be estimated as described above. For example, the likelihood of microbubbles may be determined as a function of water content in the insulation based on the relationship described in Oommen et al. "Bubble Evolution from Transformer Overload," doi:10.1109/TDC.2001.971223.

In an alternative or additional embodiment of subprocess 480, the at least one parameter comprises a breakdown voltage of insulating liquid 360. This breakdown voltage may be estimated based on the total water content in insulating liquid 360, as determined by measurement (e.g., by sensor 370) and/or based on the total water content in electrical terminal 150, which may be estimated as described above. For example, the breakdown voltage may be determined as a function of water content in insulating liquid 360 based on the relationship described in Balma.

Regardless of which parameter(s) are estimated in subprocess 480, the parameter(s) estimated in subprocess 480 may be used in any beneficial manner. For example, the parameter(s) may be provided in a report (e.g., provided in a graphical user interface on a display of user system 130). The report may indicate any of the described parameters (e.g., estimated water content in insulating liquid 360, estimated water content in the insulation, estimated total water content in electrical transformer 150, measure of aging, risk of dielectric failure, likelihood of microbubbles, breakdown voltage, etc.), as well as any parameters derived from the indicated parameters, which may represent risk of operation, estimated lifespan, and/or the like. Such a report may be used by an operator of electrical transformer 150 to schedule or otherwise plan for maintenance of electrical transformer 150, replacement of electrical transformer 150, increased or reduced loading on electrical transformer 150, and/or the like.

Alternatively or additionally, the parameter(s) may be used to generate one or more alerts. For instance, when a parameter, such as the risk of dielectric failure, likelihood of microbubbles, or breakdown voltage, satisfies a threshold (e.g., risk of dielectric failure exceeds a predefined threshold, the likelihood of microbubbles exceeds a predefined threshold, or the breakdown voltage falls below a predefined threshold), platform 110 may generate an alert to at least one recipient, which may be responsible personnel or another system. The alert may comprise a communication (e.g., email message, pre-recorded or synthesized telephone message, Short Message Service (SMS) or Multimedia Messaging Service (MMS) message, an intra-application message within server application 112, an inter-application message via an API, etc.), for example, to one or more user accounts or user system(s) 130 or other systems.

Alternatively or additionally, the parameter(s) may be used to trigger one or more physical controls. For instance, when a parameter, such as the risk of dielectric failure, likelihood of microbubbles, or breakdown voltage, satisfies a threshold (e.g., risk of dielectric failure exceeds a predefined threshold, the likelihood of microbubbles exceeds a predefined threshold, or the breakdown voltage falls below a predefined threshold), indicating unsafe operation, platform 110 may automatically trip electrical transformer 150, limit the loading capacity of electrical transformer, or control some other related component of a power network. However, it should be understood that this may not warranted or permissible in scenarios in which the control would have significant consequences (e.g., severe power outage). In such cases, platform 110 may instead generate an alert, as described above, so that an operator may make the decision regarding whether or not to implement a control.

For the purposes of validation of disclosed embodiments, an implementation of process 400 was executed on a simulated electrical transformer 150. In the tested implementation, process 400 was executed for a plurality of time intervals, using the water content in insulating liquid 360 as the only independent variable. In each iteration of the outer loop of process 400, a solver was used to find the water content in insulating liquid 360 that resulted in a total water content inside electrical transformer 150 that matches the value at the prior iteration of the outer loop of process 400, plus or minus the value of the adjustment parameter. In particular, for each time interval, the solver calculates a new transient condition for the temperature and distribution of water content that satisfies the equations described herein and produces the total water content inside electrical transformer 150 within an error tolerance. At the end of each time interval, the water content inside electrical transformer 150 must match the water content at the beginning of the time interval plus the water content generated by the degradation of the insulation. In other words, the system of non-linear, interconnected equations, described herein, are integrated into a single target function that is optimized by the solver, using the water content in insulating liquid 360 as the independent variable.

The simulated electrical transformer 150 was a 25 Megavolt-amperes (MVA) transformer, having a single coil 350, and subjected to nominal conditions. The simulated electrical transformer 150 had a hot-spot temperature rise of 80°C, reaching 110°C for a flat ambient temperature of 30°C. The initial conditions for the simulated electrical transformer 150 were set as 0.5% water content in the insulation and 10 ppm of water in insulating liquid 360. The simulated electrical transformer 150 was assumed to be perfectly sealed. After a settling time of 168 hours (7 days) at ambient temperature, the simulated electrical transformer 150 was energized and continuously maintained at the rated capacity. The ambient temperature was set to 30°C flat, to provide nominal life conditions for the insulation degradation. The following parameters were used: Δ*θ_{TO,R}* = 49°C; vertical temperature gradient=20°C; winding-to-oil temperature gradient=20°C; hot-spot factor=1.4; *n* = 0.8; *R* = 9.5; core and coils weight=50,000 kilograms (kg); tank and accessories weight=15,000 kg; volume of mineral oil as insulating liquid 360=70,000 liters; and total losses = 277.423 kilowatts (kW).

Notably, during the settling time, the simulated electrical transformer 150 was deenergized, such that the temperature is relatively low at or near the ambient temperature. This led to an initial intense migration of water from insulating liquid 360 towards the insulation, since the relative saturation of the insulation with 0.5% water content is lower than that of mineral oil having 10 ppm of water. In particular, the water content in insulating liquid 360 dropped from 10 ppm to less than 2 ppm. This only increased the water content in the insulation from 0.5% to 0.54%, since the water absorption capacity of the insulation largely exceeded that of insulating liquid 360. However, this trend was reversed when the simulated electrical transformer 150 was energized and the temperature increased.

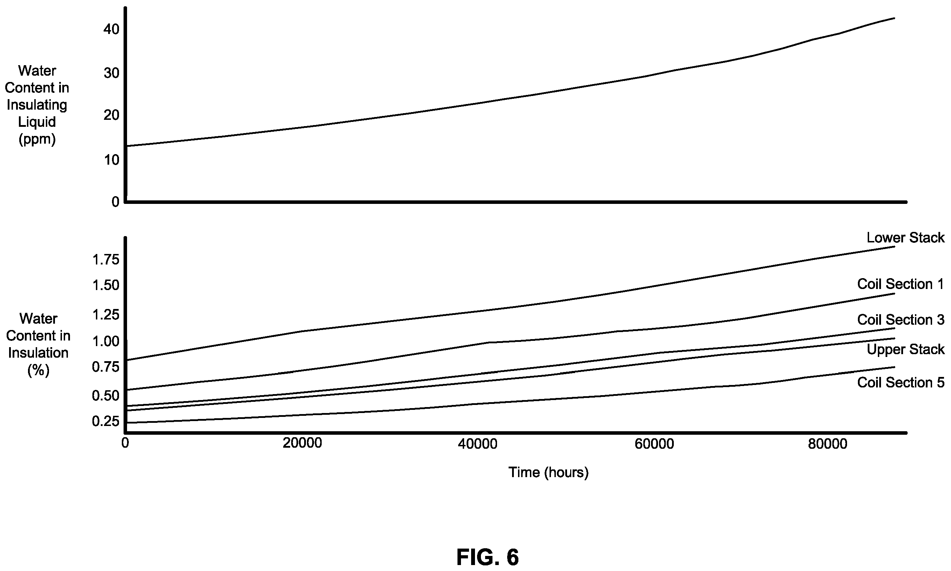

FIG. 6 illustrates the water content in insulating liquid 360 and the insulation over the full simulation time of 87,600 hours (10 years), according to the tested implementation of an embodiment. The water generated by the aging of the insulation remains in the system, accumulating both in insulating liquid 360 and in the insulation. Components exposed to lower temperatures (e.g., lower stack(s) 320 and lower coil sections 355) reached a water content significantly higher than the hot-spot (e.g., upper stack(s) 330 and upper coil sections 355). The driest region is the hot-spot region (e.g., the topmost coil section 355, which is coil section 5 in FIG. 6), where the water content was reduced to 0.25 after the settling time and energization, and increased to 0.75% after 10 years of operation. During the same time period, the water content in lower stack(s) 320, which represents thicker pressboard components, increased from 0.5% to 0.8% during the settling time, and increased to 1.8% after 10 years of operation. Notably, the water, generated by cellulose degradation in the insulation, increased the total water content in the simulated electrical transformer 150.

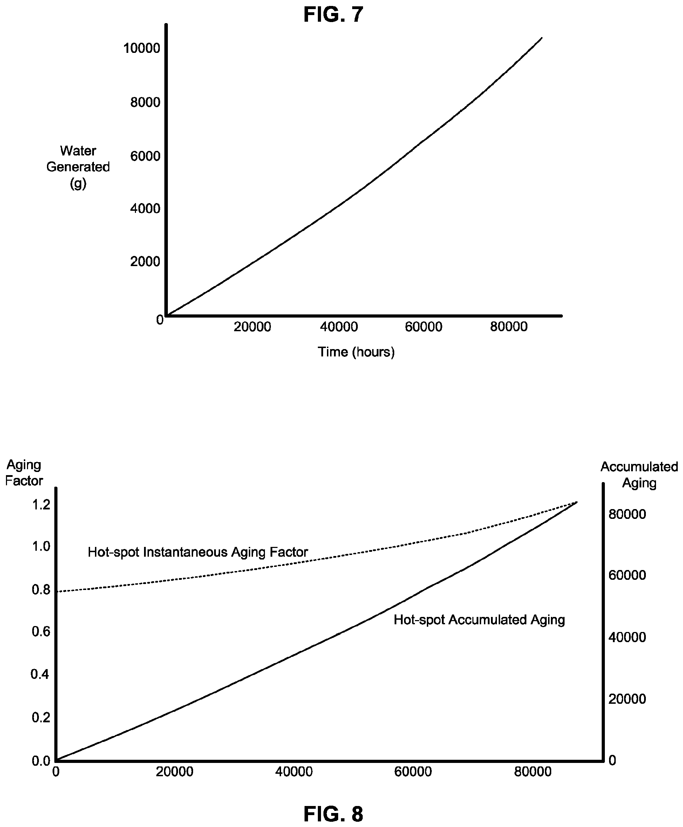

FIG. 7 illustrates the water generation, from cellulose degradation, over the full simulation time, according to the tested implementation of an embodiment. As illustrated, during the full simulation time of 10 years, the cellulose degradation generated 10.39 kg of water from 1,100 kg of insulation. Notably, the rate of water generation (i.e., cellulose degradation) accelerates as water content increases.

FIG. 8 illustrates the measure of aging over the full simulation time, according to the tested implementation of an embodiment. Both the instantaneous aging factor (i.e., *A*/*Aᵣ* ) and the accumulated aging (i.e., *V*) for the hot-spot section (e.g., the topmost coil section 355) are illustrated. Despite being the driest region, the hot-spot section reached the highest value of accumulated aging due to the higher temperature. After 87,600 hours (10 years) of operation under nominal conditions, the accumulated life consumption of the simulated electrical transformer 150 reached 83,670 hours, or 95.51% of nominal life. The calculated aging factor started at 0.8x and ended slightly over 1.2x.

Although not illustrated, during the second decade of simulation, the water content in the hot-spot section reached 1.4%. As a result, the equivalent aging of 180,000 hours was reached after 154,828 hours of operation. In other words, the effective life of the simulated electrical transformer 150 was 14% lower than the unit of life, when considering the water generation from cellulose degradation in the insulation. This demonstrates that the effective life of an electrical transformer 150, which runs continuously at the rated capacity, will be shorter than the nominal life when no drying interventions are performed. Based on the simulation, the water content in insulating liquid 360 should trigger a maintenance intervention around the twelfth year of operation, when it exceeded 25 ppm. Drying the electrical transformer 150 at this time would prevent the reduction in lifespan, and ensure that the nominal lifespan would be exceeded. In a similar manner, the disclosed embodiments may be used to schedule or otherwise plan maintenance interventions for any electrical transformer 150.

As discussed above, it is not possible to place sensors within electrical transformer 150 to measure the degradation of the insulation during operation of the electrical transformer 150. Thus, traditionally, the electrical transformer 150 must be shut down and opened to determine the degradation of the insulation, resulting in disruptions to operations. Process 400 obviates such disruptions by continuously and iteratively estimating the water content in various sections of electrical transformer 150, based on the known initial conditions and the output of moisture and temperature sensors (e.g., 370). Thus, disclosed embodiments can emulate a sensor within electrical transformer 150 in a manner that does not require destructive testing. It should be understood that the disclosed embodiments may be incorporated into a larger digital twin of electrical transformer 150, which models one or more other aspects of electrical transformer 150.

## Claims

1. A method for modeling thermochemical degradation in a digital twin of an electrical transformer, the method comprising using at least one hardware processor to, for each of one or more time intervals:
determine a total water content in insulating liquid of the electrical transformer;
for each of a plurality of sections of at least one winding in the electrical transformer, logically divided along at least one axis of the at least one winding,
estimate a temperature of the section based on a loading of the electrical transformer and a temperature gradient along the at least one axis,
estimate a diffusion-related water content in insulation of the at least one winding in the section, resulting from diffusion, based on the estimated temperature and the total water content in the insulating liquid,
estimate a degradation-related water content, based on degradation of a material of the insulation, and
estimate a total water content within the insulation of the at least one winding in the section based on the diffusion-related water content and the degradation-related water content; and
estimate a total water content in the electrical transformer based on the total water content in the insulating liquid and the total water content within the insulation of the at least one winding in all of the plurality of sections.

2. The method of Claim 1, further comprising using the at least one hardware processor to, for each of the one or more time intervals, determine an average temperature of the insulating liquid for each of a plurality of time instants spanning the time interval.

3. The method of Claim 2, wherein the plurality of sections comprises:
at least one lower stack that represents a first assembling element at a first end of the at least one winding along the longitudinal axis;
at least one upper stack that represents a second assembling element at a second end of the at least one winding that is opposite the first end of the at least one winding along the longitudinal axis; and
one or more coil sections that each represents at least a portion of at least one coil between the first end and the second end of the at least one winding.

4. The method of Claim 3, wherein the temperature gradient comprises an axial temperature gradient along an axial axis and a radial temperature gradient along a radial axis, and wherein estimating the temperature of each of the plurality of sections comprises, for each of the plurality of time instants:
calculating a localized temperature of the at least one lower stack, based on the average temperature of the insulating liquid and the axial temperature gradient calculated for the time instant;
calculating a localized temperature of the at least one upper stack based on the average temperature of the insulating liquid and the axial temperature gradient calculated for the time instant;
and calculating a localized temperature for each of the one or more coil sections, based on the temperature of the at least one lower stack or at least one upper stack, the loading, and the radial and axial temperature gradients.

5. The method of Claim 4, wherein the one or more coils sections are a plurality of coil sections, and wherein the temperature for one of the plurality of coil sections that is closest to the second end is calculated further based on a hot-spot factor or heat-dissipation factor associated with the electrical transformer.

6. The method of Claim 1, wherein estimating the diffusion-related water content comprises, for each of a plurality of time instants spanning the time interval, calculating a water content diffused into the insulation based on a time constant for diffusion of water into the material of the insulation.

7. The method of Claim 6, wherein, for each of the plurality of time instants, the water content diffused into the insulation is calculated as: ${W}_{diffusion} = \left({W}_{equilibrium}-{W}_{initial}\right) ⋅ \left(1-{e}^{- \frac{t}{{τ}_{p}}}\right) + {W}_{initial}$ wherein *W_{diffusion}* is the water content diffused into the insulation at time instant *t, W_{equilibrium}* is a water content in the insulation at an equilibrium condition, *Wᵢₙᵢₜᵢₐₗ* is a water content in the insulation at a time instant immediately preceding time instant *t, e* is Euler's number, and *τₚ* is the time constant.

8. The method of Claim 1, wherein the material of the insulation is cellulose-based.

9. The method of Claim 8, wherein the degradation-related water content in the insulation is estimated based on a water generation rate from scissions of cellulose chains in the cellulose-based material.

10. The method of Claim 9, wherein the water generation rate is calculated as: ${W}_{rate} = {\int }_{0}^{k} {2}^{x + 1} dx ⋅ \frac{{Mol}_{water}}{DP ⋅ {Mol}_{glucose}} ⋅ \frac{1}{180 , 000}$ wherein *Wᵣₐₜₑ* is an amount of water generated per hour, *k* is a number of scissions, *Mol_{water}* is a molar mass of water, *DP* is a degree of polymerization of the material of the insulation, and *Mol_{glucose}* is a molar mass of a monomer of glucose.

11. The method of Claim 1, further comprising using the at least one hardware processor to, for each of the one or more time intervals,
for each of the plurality of sections of the at least one winding in the electrical transformer, calculate a measure of aging for the section based on the total water content in the insulation; and
determine an overall measure of aging of the electrical transformer based on the measures of aging for the plurality of sections.

12. The method of Claim 11, wherein the measure of aging for each of the plurality of sections is further based on one or both of an oxygen content in the insulation or an acidity of the insulating liquid.

13. The method of Claim 11, wherein calculating the measure of aging for each of the plurality of sections comprises:
determining a rated aging parameter associated with the material of the insulation;
interpolating an aging parameter of the section based on the total water content in the insulation of the at least one winding in the section; and
calculating an aging ratio between the rated aging parameter and the interpolated aging parameter.

14. The method of Claim 13, wherein calculating the measure of aging for each of the plurality of sections further comprises:
determining an activation energy at rated conditions associated with the material of the insulation;
interpolating an activation energy of the material of the insulation;
calculating an exponentiation factor based on the activation energy at rated conditions and the interpolated activation energy; and
determining the measure of aging for the section based on the aging ratio and the exponentiation factor.

15. The method of Claim 14, wherein the measure of aging for each of the plurality of sections is calculated as: $V = \frac{A}{{A}_{r}} ⋅ {e}^{\frac{1}{R} \times \left(\frac{{E}_{r}}{{θ}_{h , r} + 273}-\frac{E}{{θ}_{h} + 273}\right)}$ wherein *V* is the measure of aging, *A* is the interpolated aging parameter, *Aᵣ* is the rated aging parameter, *e* is Euler's number, *R* is a molar gas constant, *Eᵣ* is the activation energy at rated conditions, *E* is the interpolated activation energy, *θ_{h,r}* is a hot-spot-to-top-oil temperature at a rated load, and *θₕ* is a hot-spot temperature.

16. The method of Claim 1, wherein determining the total water content in the insulating liquid comprises deriving the total water content in the insulating liquid from an output of at least one sensor in the electrical transformer, and wherein the method further comprises using the at least one hardware processor to, at least once for each of the one or more time intervals:
calculate a deviation between an actual value of the total water content in the insulating liquid, derived from the output of the at least one sensor, and one or more estimated values of the total water content in the insulating liquid output by an artificial intelligence (AI) model of the digital twin; and
determine an adjustment parameter based on the deviation, wherein the estimate of the total water content in the transformer is further based on the adjustment parameter.

17. The method of Claim 1, further comprising using the at least one hardware processor to estimate a risk of dielectric failure of the insulation of the at least one winding based on the estimated total water content within the insulation.

18. The method of Claim 1, further comprising using the at least one hardware processor to estimate a likelihood of microbubbles on a surface of the insulation of the at least one winding based on the estimated total water content within the insulation.

19. The method of Claim 1, further comprising using the at least one hardware processor to:
estimate a breakdown voltage of the insulating liquid based on the total water content in the insulating liquid; and
when the estimated breakdown voltage satisfies a threshold, generate an alert.

20. A system comprising:
at least one hardware processor; and
software configured to, when executed by the at least one hardware processor, model thermochemical degradation in a digital twin of an electrical transformer by, for each of one or more time intervals,
determine a total water content in insulating liquid of the electrical transformer,
for each of a plurality of sections of at least one winding in the electrical transformer, logically divided along at least one axis of the at least one winding,
estimate a temperature of the section based on a loading of the electrical transformer and a temperature gradient along the at least one axis,
estimate a diffusion-related water content in insulation of the at least one winding in the section, resulting from diffusion, based on the estimated temperature and the total water content in the insulating liquid,
estimate a degradation-related water content, based on degradation of a material of the insulation, and
estimate a total water content within the insulation of the at least one winding in the section based on the diffusion-related water content and the degradation-related water content, and
estimate a total water content in the transformer based on the total water content in the insulating liquid and the total water content within the insulation of the at least one winding in all of the plurality of sections.

## Patentansprüche

1. Verfahren zum Modellieren von thermochemischem Abbau in einem digitalen Zwilling eines elektrischen Transformators, wobei das Verfahren Verwenden mindestens eines Hardwareprozessors umfasst, um für jedes von einem oder mehreren Zeitintervallen Folgendes durchzuführen:
Bestimmen eines Gesamtwassergehalts in Isolierflüssigkeit des elektrischen Transformators;
für jeden von einer Vielzahl von Abschnitten mindestens einer Wicklung in dem elektrischen Transformator, die logisch entlang mindestens einer Achse der mindestens einen Wicklung geteilt sind,
Schätzen einer Temperatur des Abschnitts basierend auf einer Belastung des elektrischen Transformators und einem Temperaturgradienten entlang der mindestens einen Achse, Schätzen eines diffusionsbezogenen Wassergehalts bei der Isolierung der mindestens einen Wicklung in dem Abschnitt, der aus Diffusion resultiert, basierend auf der geschätzten Temperatur und dem Gesamtwassergehalt in der Isolierflüssigkeit,
Schätzen eines abbaubezogenen Wassergehalts basierend auf Abbau eines Materials der Isolierung, und
Schätzen eines Gesamtwassergehalts innerhalb der Isolierung der mindestens einen Wicklung in dem Abschnitt basierend auf dem diffusionsbezogenen Wassergehalt und dem abbaubezogenen Wassergehalt; und
Schätzen eines Gesamtwassergehalts in dem elektrischen Transformator basierend auf dem Gesamtwassergehalt in der Isolierflüssigkeit und dem Gesamtwassergehalt innerhalb der Isolierung der mindestens einen Wicklung in allen der Vielzahl von Abschnitten.

2. Verfahren nach Anspruch 1, ferner umfassend Verwenden des mindestens einen Hardwareprozessors zum Bestimmen einer durchschnittlichen Temperatur der Isolierflüssigkeit für jeden einer Vielzahl von Zeitpunkten, die das Zeitintervall überspannen, für jedes des einen oder der mehreren Zeitintervalle.

3. Verfahren nach Anspruch 2, wobei die Vielzahl von Abschnitten umfasst:
mindestens einen unteren Stapel, der ein erstes Anordnungselement repräsentiert, an einem ersten Ende der mindestens einen Wicklung entlang der Längsachse;
mindestens einen oberen Stapel, der ein zweites Anordnungselement repräsentiert, an einem zweiten Ende der mindestens einen Wicklung, das dem ersten Ende der mindestens einen Wicklung entlang der Längsachse gegenüberliegt; und
einen oder mehrere Spulenabschnitte, die jeweils mindestens einen Anteil mindestens einer Spule zwischen dem ersten Ende und dem zweiten Ende der mindestens einen Wicklung repräsentieren.

4. Verfahren nach Anspruch 3, wobei der Temperaturgradient einen axialen Temperaturgradienten entlang einer axialen Achse und einen radialen Temperaturgradienten entlang einer radialen Achse umfasst, und wobei Schätzen der Temperatur von jedem der Vielzahl von Abschnitten für jeden der Vielzahl von Zeitpunkten umfasst:
Berechnen einer lokalisierten Temperatur des mindestens einen unteren Stapels basierend auf der durchschnittlichen Temperatur der Isolierflüssigkeit und dem für den Zeitpunkt berechneten axialen Temperaturgradienten;
Berechnen einer lokalisierten Temperatur des mindestens einen oberen Stapels basierend auf der durchschnittlichen Temperatur der Isolierflüssigkeit und dem axialen Temperaturgradienten, die für den Zeitpunkt berechnet werden;
und Berechnen einer lokalisierten Temperatur für jeden des einen oder der mehreren Spulenabschnitte basierend auf der Temperatur des mindestens einen unteren Stapels oder mindestens einen oberen Stapels, der Belastung und den radialen und axialen Temperaturgradienten.

5. Verfahren nach Anspruch 4, wobei der eine oder die mehreren Spulenabschnitte eine Vielzahl von Spulenabschnitten sind, und wobei die Temperatur für einen der Vielzahl von Spulenabschnitten, der dem zweiten Ende am nächsten ist, ferner basierend auf einem Hotspot-Faktor oder Wärmeableitungsfaktor berechnet wird, der mit dem elektrischen Transformator assoziiert ist.

6. Verfahren nach Anspruch 1, wobei Schätzen des diffusionsbezogenen Wassergehalts für jeden von der Vielzahl von Zeitpunkten, die das Zeitintervall überspannen, Berechnen eines in die Isolierung diffundierten Wassergehalts basierend auf einer Zeitkonstante für die Diffusion von Wasser in das Material der Isolierung hinein umfasst.

7. Verfahren nach Anspruch 6, wobei für jeden der Vielzahl von Zeitpunkten der in die Isolierung diffundierte Wassergehalt berechnet wird als: ${W}_{Diffusion} = \left({W}_{Gleichgewicht}-{W}_{anf ä nglich}\right) ⋅ \left(1-{e}^{- \frac{t}{{τ}_{p}}}\right) + {W}_{anf ä nglich}$ wobei W_{Diffusion} der zum Zeitpunkt t in die Isolierung diffundierte Wassergehalt ist, W_{Gleichgewicht} ein Wassergehalt in der Isolierung bei einem Gleichgewichtszustand ist, W_{anfänglich} ein Wassergehalt in der Isolierung zu einem Zeitpunkt ist, der dem Zeitpunkt t unmittelbar vorausgeht, e die Eulersche Zahl ist, und τₚ die Zeitkonstante ist.

8. Verfahren nach Anspruch 1, wobei das Material der Isolierung auf Cellulose basiert.

9. Verfahren nach Anspruch 8, wobei der abbaubezogene Wassergehalt in der Isolierung basierend auf einer Wassererzeugungsrate aus Spaltung von Celluloseketten in dem Material auf Cellulosebasis geschätzt wird.

10. Verfahren nach Anspruch 9, wobei die Wassererzeugungsrate berechnet wird als: ${W}_{Rate} = {\int }_{0}^{k} {2}^{x + 1} dx ⋅ \frac{{Mol}_{Wasser}}{DP ⋅ {Mol}_{Glucose}} ⋅ \frac{1}{180.000}$ wobei W_{Rate} eine Menge an pro Stunde erzeugtem Wasser ist, k eine Anzahl von Spaltungen ist, Mol_{Wasser} eine Molmasse von Wasser ist, DP ein Polymerisationsgrad des Materials der Isolierung ist und Mol_{Glucose} eine Molmasse eines Monomers von Glucose ist.

11. Verfahren nach Anspruch 1, ferner umfassend Verwenden des mindestens einen Hardwareprozessors zum Durchführen von Folgendem für jedes des einen oder der mehreren Zeitintervalle:
für jeden der Vielzahl von Abschnitten der mindestens einen Wicklung in dem elektrischen Transformator, Berechnen eines Alterungsmaßes für den Abschnitt basierend auf dem Gesamtwassergehalt in der Isolierung; und
Bestimmen eines Gesamtalterungsmaßes des elektrischen Transformators basierend auf den Alterungsmaßen für die Vielzahl von Abschnitten.

12. Verfahren nach Anspruch 11, wobei das Alterungsmaß für jeden der Vielzahl von Abschnitten ferner auf einem oder beiden von einem Sauerstoffgehalt in der Isolierung oder einer Acidität der Isolierflüssigkeit basiert.

13. Verfahren nach Anspruch 11, wobei Berechnen des Alterungsmaßes für jeden der Vielzahl von Abschnitten umfasst:
Bestimmen eines Nennalterungsparameters, der mit dem Material der Isolierung assoziiert ist;
Interpolieren eines Alterungsparameters des Abschnitts basierend auf dem Gesamtwassergehalt in der Isolierung der mindestens einen Wicklung in dem Abschnitt; und
Berechnen eines Alterungsverhältnisses zwischen dem Nennalterungsparameter und dem interpolierten Alterungsparameter.

14. Verfahren nach Anspruch 13, wobei Berechnen des Alterungsmaßes für jeden der Vielzahl von Abschnitten ferner umfasst:
Bestimmen einer Aktivierungsenergie bei Nennbedingungen, die mit dem Material der Isolierung assoziiert ist;
Interpolieren einer Aktivierungsenergie des Materials der Isolierung;
Berechnen eines Potenzierungsfaktors basierend auf der Aktivierungsenergie bei Nennbedingungen und der interpolierten Aktivierungsenergie; und
Bestimmen des Alterungsmaßes für den Abschnitt basierend auf dem Alterungsverhältnis und dem Potenzierungsfaktor.

15. Verfahren nach Anspruch 14, wobei das Alterungsmaß für jeden der Vielzahl von Abschnitten berechnet wird als: $V = \frac{A}{{A}_{r}} ⋅ {e}^{\frac{1}{R} \times \left(\frac{{E}_{r}}{{θ}_{h , r} + 273}-\frac{E}{{θ}_{h} + 273}\right)}$ wobei V das Alterungsmaß ist, A der interpolierte Alterungsparameter ist, Aᵣ der Nennalterungsparameter ist, e die Eulersche Zahl ist, R eine molare Gaskonstante ist, Eᵣ die Aktivierungsenergie bei Nennbedingungen ist, E die interpolierte Aktivierungsenergie ist, θ_{h,r} eine Hotspot-to-Top-Öltemperatur bei einer Nennbelastung ist, und θₕ eine Hotspot-Temperatur ist.

16. Verfahren nach Anspruch 1, wobei Bestimmen des Gesamtwassergehalts in der Isolierflüssigkeit Ableiten des Gesamtwassergehalts in der Isolierflüssigkeit von einer Ausgabe von mindestens einem Sensor in dem elektrischen Transformator umfasst, und wobei das Verfahren ferner Verwenden des mindestens einen Hardwareprozessors umfasst, um mindestens einmal für jedes des einen oder der mehreren Zeitintervalle Folgendes durchzuführen:
Berechnen einer Abweichung zwischen einem tatsächlichen Wert des Gesamtwassergehalts in der Isolierflüssigkeit, der von der Ausgabe des mindestens einen Sensors abgeleitet ist, und einem oder mehreren geschätzten Werten des Gesamtwassergehalts in der Isolierflüssigkeit, ausgegeben durch ein künstliches Intelligenz(AI)-Modell des digitalen Zwillings; und
Bestimmen eines Anpassungsparameters basierend auf der Abweichung, wobei die Schätzung des Gesamtwassergehalts in dem Transformator ferner auf dem Anpassungsparameter basiert.

17. Verfahren nach Anspruch 1, ferner umfassend Verwenden des mindestens einen Hardwareprozessors zum Schätzen eines Risikos eines dielektrischen Ausfalls der Isolierung der mindestens einen Wicklung basierend auf dem geschätzten Gesamtwassergehalt innerhalb der Isolierung.

18. Verfahren nach Anspruch 1, ferner umfassend Verwenden des mindestens einen Hardwareprozessors zum Schätzen einer Wahrscheinlichkeit von Mikroblasen auf einer Oberfläche der Isolierung der mindestens einen Wicklung basierend auf dem geschätzten Gesamtwassergehalt innerhalb der Isolierung.

19. Verfahren nach Anspruch 1, ferner umfassend Verwenden des mindestens einen Hardwareprozessors zum:
Schätzen einer Durchbruchspannung der Isolierflüssigkeit basierend auf dem Gesamtwassergehalt in der Isolierflüssigkeit; und
Generieren eines Alarms, wenn die geschätzte Durchbruchspannung einen Schwellenwert erfüllt.

20. System, umfassend:
mindestens einen Hardwareprozessor; und
Software, die dazu ausgelegt ist, bei Ausführung durch den mindestens einen Hardwareprozessor thermochemischen Abbau in einem digitalen Zwilling eines elektrischen Transformators zu modellieren, indem für jedes von einem oder mehreren Zeitintervallen Folgendes durchgeführt wird:
Bestimmen eines Gesamtwassergehalts in Isolierflüssigkeit des elektrischen Transformators,
für jeden von einer Vielzahl von Abschnitten mindestens einer Wicklung in dem elektrischen Transformator, die logisch entlang mindestens einer Achse der mindestens einen Wicklung geteilt sind,
Schätzen einer Temperatur des Abschnitts basierend auf einer Belastung des elektrischen Transformators und einem Temperaturgradienten entlang der mindestens einen Achse, Schätzen eines diffusionsbezogenen Wassergehalts bei der Isolierung der mindestens einen Wicklung in dem Abschnitt, der aus Diffusion resultiert, basierend auf der geschätzten Temperatur und dem Gesamtwassergehalt in der Isolierflüssigkeit,
Schätzen eines abbaubezogenen Wassergehalts basierend auf Abbau eines Materials der Isolierung, und
Schätzen eines Gesamtwassergehalts innerhalb der Isolierung der mindestens einen Wicklung in dem Abschnitt basierend auf dem diffusionsbezogenen Wassergehalt und dem abbaubezogenen Wassergehalt, und
Schätzen eines Gesamtwassergehalts in dem Transformator basierend auf dem Gesamtwassergehalt in der Isolierflüssigkeit und dem Gesamtwassergehalt innerhalb der Isolierung der mindestens einen Wicklung in allen der Vielzahl von Abschnitten.

## Revendications

1. Procédé destiné à modéliser la dégradation thermochimique dans un jumeau numérique d'un transformateur électrique, le procédé comprenant l'utilisation d'au moins un processeur matériel pour, pour chacun d'un ou plusieurs intervalles de temps :
déterminer une teneur totale en eau dans un liquide isolant du transformateur électrique ;
pour chaque section d'une pluralité de sections d'au moins un enroulement dans le transformateur électrique, divisé logiquement le long d'au moins un axe de l'au moins un enroulement,
estimer une température de la section en fonction d'une charge du transformateur électrique et d'un gradient de température le long de l'au moins un axe,
estimer une teneur en eau associée à la diffusion dans l'isolation de l'au moins un enroulement de la section, résultant de la diffusion, en fonction de la température estimée et de la teneur totale en eau dans le liquide isolant,
estimer une teneur en eau associée à la dégradation, en fonction de la dégradation d'un matériau de l'isolation, et
estimer une teneur totale en eau à l'intérieur de l'isolation de l'au moins un enroulement dans la section en fonction de la teneur en eau associée à la diffusion et de la teneur en eau associée à la dégradation ; et
estimer une teneur totale en eau dans le transformateur électrique en fonction de la teneur totale en eau dans le liquide isolant et de la teneur totale en eau à l'intérieur de l'isolation de l'au moins un enroulement dans toutes les sections de la pluralité de sections.

2. Procédé de la revendication 1, comprenant en outre l'utilisation de l'au moins un processeur matériel pour, pour l'intervalle de temps ou chacun des intervalles de temps, déterminer une température moyenne du liquide isolant pour chaque instant d'une pluralité d'instants couvrant l'intervalle de temps.

3. Procédé de la revendication 2, dans lequel la pluralité de sections comprend :
au moins un empilement inférieur qui représente un premier élément d'assemblage à une première extrémité de l'au moins un enroulement le long de l'axe longitudinal ;
au moins un empilement supérieur qui représente un deuxième élément d'assemblage à une deuxième extrémité de l'au moins un enroulement qui se situe à l'opposé de la première extrémité de l'au moins un enroulement le long de l'axe longitudinal ; et
une ou plusieurs sections de bobine qui représentent chacune au moins une partie d'au moins une bobine entre la première extrémité et la deuxième extrémité de l'au moins un enroulement.

4. Procédé de la revendication 3, dans lequel le gradient de température comprend un gradient de température axial le long d'un axe axial et un gradient de température radial le long d'un axe radial, et dans lequel l'estimation de la température de chaque section de la pluralité de sections comprend, pour chaque instant de la pluralité d'instants :
le calcul d'une température localisée de l'au moins un empilement inférieur, en fonction de la température moyenne du liquide isolant et du gradient de température axial calculé pour l'instant ;
le calcul d'une température localisée de l'au moins un empilement supérieur, en fonction de la température moyenne du liquide isolant et du gradient de température axial calculé pour l'instant ;
et le calcul d'une température localisée pour la section ou chacune des sections de bobine, en fonction de la température de l'au moins un empilement inférieur ou de l'au moins un empilement supérieur, de la charge, et des gradients de température radial et axial.

5. Procédé de la revendication 4, dans lequel la ou les sections de bobine sont une pluralité de sections de bobine, et dans lequel la température pour une section de la pluralité de sections de bobine qui est la plus proche de la deuxième extrémité est également calculée en fonction d'un facteur de point chaud ou d'un facteur de dissipation thermique associé au transformateur électrique.

6. Procédé de la revendication 1, dans lequel l'estimation de la teneur en eau associée à la diffusion comprend, pour chaque instant d'une pluralité d'instants couvrant l'intervalle de temps, le calcul d'une teneur en eau ayant diffusé à l'intérieur de l'isolation en fonction d'une constante de temps pour la diffusion de l'eau à l'intérieur du matériau de l'isolation.

7. Procédé de la revendication 6 dans lequel, pour chaque instant de la pluralité d'instants, la teneur en eau ayant diffusé à l'intérieur de l'isolation est calculée comme suit : ${W}_{diffusion} = \left({W}_{equilibrium}-{W}_{initial}\right) ⋅ \left(1-{e}^{- \frac{t}{{τ}_{p}}}\right) + {W}_{initial}$ où W_{diffusion} est la teneur en eau ayant diffusé à l'intérieur de l'isolation à l'instant t, W_{equilibrium} est une teneur en eau dans l'isolation dans un état d'équilibre, Wᵢₙᵢₜᵢₐₗ est une teneur en eau dans l'isolation à un instant juste avant l'instant t, e est le nombre d'Euler, et τₚ est la constante de temps.

8. Procédé de la revendication 1, dans lequel le matériau de l'isolation est à base de cellulose.

9. Procédé de la revendication 8, dans lequel la teneur en eau associée à la dégradation dans l'isolation est estimée en fonction d'un taux de production d'eau à partir de scissions de chaînes cellulosiques dans le matériau à base de cellulose.

10. Procédé de la revendication 9, dans lequel le taux de production d'eau est calculé comme suit : ${W}_{Rate} = {\int }_{0}^{k} {2}^{x + 1} dx ⋅ \frac{{Mol}_{water}}{DP ⋅ {Mol}_{glucose}} ⋅ \frac{1}{180 000}$ où Wᵣₐₜₑ est une quantité d'eau produite par heure, k est un nombre de scissions, Mol_{water} est une masse molaire d'eau, DP est un degré de polymérisation du matériau de l'isolation, et Mol_{glucose} est une masse molaire d'un monomère du glucose.

11. Procédé de la revendication 1, comprenant en outre l'utilisation de l'au moins un processeur matériel pour, pour l'intervalle de temps ou chacun des intervalles de temps :
pour chaque section de la pluralité de sections de l'au moins un enroulement dans le transformateur électrique, calculer une mesure du vieillissement pour la section en fonction de la teneur totale en eau dans l'isolation ; et
déterminer une mesure globale du vieillissement du transformateur électrique en fonction des mesures de vieillissement pour la pluralité de sections.

12. Procédé de la revendication 11, dans lequel la mesure du vieillissement pour chaque section de la pluralité de sections est également basée sur une teneur en oxygène dans l'isolation et/ou une acidité du liquide isolant.

13. Procédé de la revendication 11, dans lequel le calcul de la mesure du vieillissement pour chaque section de la pluralité de sections comprend :
la détermination d'un paramètre de vieillissement nominal associé au matériau de l'isolation ;
l'interpolation d'un paramètre de vieillissement de la section en fonction de la teneur totale en eau dans l'isolation de l'au moins un enroulement dans la section ; et
le calcul d'un rapport de vieillissement entre le paramètre de vieillissement nominal et le paramètre de vieillissement interpolé.

14. Procédé de la revendication 13, dans lequel le calcul de la mesure du vieillissement pour chaque section de la pluralité de sections comprend en outre :
la détermination d'une énergie d'activation dans des conditions nominales associée au matériau de l'isolation ;
l'interpolation d'une énergie d'activation du matériau de l'isolation ;
le calcul d'un facteur d'exponentiation en fonction de l'énergie d'activation dans des conditions nominales et de l'énergie d'activation interpolée ; et
la détermination de la mesure du vieillissement pour la section en fonction du rapport de vieillissement et du facteur d'exponentiation.

15. Procédé de la revendication 14, dans lequel la mesure du vieillissement pour chaque section de la pluralité de sections est calculée comme suit : $V = \frac{A}{{A}_{r}} ⋅ {e}^{\frac{1}{R} \times \left(\frac{{E}_{r}}{{θ}_{h , r} + 273}-\frac{E}{{θ}_{h} + 273}\right)}$ où V est la mesure du vieillissement, A est le paramètre de vieillissement interpolé, Aᵣ est le paramètre de vieillissement nominal, e est le nombre d'Euler, R est une constante molaire des gaz, Eᵣ est l'énergie d'activation dans des conditions nominales, E est l'énergie d'activation interpolée, θ_{h,R} est une température entre point chaud et huile supérieure sous une charge nominale, et θₕ est une température de point chaud.

16. Procédé de la revendication 1, la détermination de la teneur totale en eau dans le liquide isolant comprenant la déduction de la teneur totale en eau dans le liquide isolant à partir d'une sortie d'au moins un capteur dans le transformateur électrique, et le procédé comprenant en outre l'utilisation de l'au moins un processeur matériel pour, au moins une fois pour l'intervalle de temps ou chacun des intervalles de temps :
calculer un écart entre une valeur réelle de la teneur totale en eau dans le liquide isolant, déduite à partir de la sortie de l'au moins un capteur, et une ou plusieurs valeurs estimées de la teneur totale en eau dans le liquide isolant restituées par un modèle d'intelligence artificielle (IA) du jumeau numérique ; et
déterminer un paramètre d'ajustement en fonction de l'écart, l'estimation de la teneur totale en eau dans le transformateur étant également basée sur le paramètre d'ajustement.

17. Procédé de la revendication 1, comprenant en outre l'utilisation de l'au moins un processeur matériel pour estimer un risque de défaillance diélectrique de l'isolation de l'au moins un enroulement en fonction de la teneur totale en eau estimée dans l'isolation.

18. Procédé de la revendication 1, comprenant en outre l'utilisation de l'au moins un processeur matériel pour estimer une probabilité de microbulles sur une surface de l'isolation de l'au moins un enroulement en fonction de la teneur totale en eau estimée à l'intérieur de l'isolation.

19. Procédé de la revendication 1, comprenant en outre l'utilisation de l'au moins un processeur matériel pour :
estimer une tension de claquage du liquide isolant en fonction de la teneur totale en eau du liquide isolant ; et
quand la tension de claquage estimée satisfait un seuil, générer une alerte.

20. Système comprenant :
au moins un processeur matériel ; et
un logiciel conçu pour, lors de son exécution par l'au moins un processeur matériel, modéliser la dégradation thermochimique dans un jumeau numérique d'un transformateur électrique en effectuant, pour chacun d'un ou plusieurs intervalles de temps, les opérations suivantes :
déterminer une teneur totale en eau dans un liquide isolant du transformateur électrique,
pour chaque section d'une pluralité de sections d'au moins un enroulement dans le transformateur électrique, divisé logiquement le long d'au moins un axe de l'au moins un enroulement,
estimer une température de la section en fonction d'une charge du transformateur électrique et d'un gradient de température le long de l'au moins un axe,
estimer une teneur en eau associée à la diffusion dans l'isolation de l'au moins un enroulement de la section, résultant de la diffusion, en fonction de la température estimée et de la teneur totale en eau dans le liquide isolant,
estimer une teneur en eau associée à la dégradation, en fonction de la dégradation d'un matériau de l'isolation, et
estimer une teneur totale en eau à l'intérieur de l'isolation de l'au moins un enroulement dans la section en fonction de la teneur en eau associée à la diffusion et de la teneur en eau associée à la dégradation, et
estimer une teneur totale en eau dans le transformateur en fonction de la teneur totale en eau dans le liquide isolant et de la teneur totale en eau à l'intérieur de l'isolation de l'au moins un enroulement dans toutes les sections de la pluralité de sections.
